# EUROPEAN PATENT APPLICATION

(11) **EP 4 696 281 A1**
(43) Date of publication of application: **18.02.2026**
(21) Application number: 25798842.8
(22) Date of filing: 14.05.2025
(51) Int. Cl.: A61C 7/00, G16H 20/30, G16H 50/50, G06T 17/30, G06F 30/20

(54) **METHOD AND APPARATUS FOR DETERMINING REFERENCE CURVE, ORTHODONTIC APPLIANCE AND DESIGN METHOD**

(30) Priority: 15.05.2024 CN 202410600135
(71) Applicant: Wuxi Ea Medical Instruments Technologies Limited, Wuxi, Jiangsu 214174 (CN)
(72) Inventor: SUN, Jiawei, Wuxi, Jiangsu 214174 (CN); WANG, Mingzheng, Wuxi, Jiangsu 214174 (CN)
(74) Representative: Winter, Brandl - Partnerschaft mbB
(86) International application number: PCT/CN2025/094885
(87) International publication number: WO 2025/237338

(57) **Abstract**

The present application discloses a method and an apparatus for determining a reference curve, a dental orthodontic appliance, and a design method. The method for determining a reference curve includes: obtaining a dental characteristic parameter of a dental arch model data; determining, based on the dental characteristic parameter, a first feature curve in a first coordinate plane and a second feature curve in a second coordinate plane, the first feature curve being a first orthodontic reference curve in a dental arch coordinate system, and the second feature curve being a dental arch curve; and adjusting the dental characteristic parameter, an adjusted dental characteristic parameter being used for determining a second orthodontic reference curve, and the adjusted dental characteristic parameter being adapted to a corresponding first feature curve and second feature curve. The method for determining an orthodontic reference curve provided by the present application may determine an orthodontic reference curve that conforms to an actual situation, may improve an accuracy and a rationality of an orthodontic treatment, and may enhance an efficiency of determining the curve and an orthodontic design.

## Description

This application claims priority to Chinese Patent Application No. 202410600135.5, filed on May 15, 2024, entitled "Method and Apparatus for Determining Reference Curves, Dental Orthodontic Appliance and Design Method", the entire content of which is incorporated herein by reference.

### TECHNICAL FIELD

The present application relates to the field of medical device technology, and particularly relates to a method and apparatus for determining reference curves, dental orthodontic appliance and design method.

### BACKGROUND

With a development of society and increasing demands for a quality of life, people are paying more and more attention to oral health maintenance. Orthodontic treatment, especially a method of using a shell-type oral appliance for orthodontic treatment, is gradually being embraced by the majority. During a orthodontic treatment process, in addition to collecting a current state of teeth, it is also necessary to determine a reference curve for orthodontic treatment based on a dental arch model in order to implement the orthodontic treatment.

In a prior art, a technical solution for fitting a dental arch curve and arranging teeth accordingly has been proposed. On one hand, the solution requires medical staff to intervene and manually adjust based on the dental arch model to obtain a suitable dental arch curve, which requires an operator to have rich experience and takes a lot of time; on the other hand, the process of adjusting to achieve the suitable dental arch curve is solely based on a single reference curve, and a determined treatment strategy may not necessarily fit an actual situation in other dimensions beyond the reference curve, thus leading to a prolonged correction cycle and causing trouble for a user.

### SUMMARY

An object of the present application is to provide a method for determining an orthodontic reference curve, to help reduce a dependence on a manual adjustment in determining the orthodontic reference curve in a prior art and improve an effect of generation of the orthodontic reference curve.

An object of the present application is to provide a method for designing a dental orthodontic appliance.

An object of the present application is to provide a display method.

An object of the present application is to provide a dental orthodontic appliance.

An object of the present application is to provide a computer storage medium.

An object of the present application is to provide an apparatus for determining an orthodontic reference curve.

An object of the present application is to provide an apparatus for designing a dental orthodontic appliance.

An object of the present application is to provide an electronic device.

To achieve one of the objects of the application, an embodiment of the present application provides a method for determining an orthodontic reference curve, comprising: obtaining dental characteristic parameters of a dental arch model data; determining a first feature curve in a first coordinate plane and a second feature curve in a second coordinate plane based on the dental characteristic parameters, the first feature curve being a first orthodontic reference curve in a dental arch coordinate system, and the second feature curve being a dental arch curve; and adjusting the dental characteristic parameters, the adjusted dental characteristic parameters being configured to determine a second orthodontic reference curve, and the adjusted dental characteristic parameters adapting to a corresponding first feature curve and a corresponding second feature curve.

Optionally, determining at least one first distance of at least one tooth based on the first feature curve and the dental characteristic parameters, the first distance being a distance of the at least one tooth relative to the first feature curve; determining a tooth to be adjusted based on the at least one first distance; and adjusting the dental characteristic parameters based on the first distance of the tooth to be adjusted.

Optionally, determining a plurality of first distances of a plurality of teeth relative to the first feature curve based on the first feature curve and the dental characteristic parameters, the first distance being a distance of a tooth relative to the first feature curve; determining a tooth satisfying a dispersion threshold condition as a tooth to be adjusted based on a dispersion degree among the plurality of first distances; or, determining a tooth to be adjusted based on sequence information of the plurality of first distances.

Optionally, determining at least one first distance of at least one tooth based on feature point coordinate information and reference point coordinate information, the feature point coordinate information being configured to indicate a feature point coordinate of the at least one tooth, and the reference point coordinate information being configured to indicate a reference point coordinate on the first feature curve corresponding to the feature point coordinate of the at least one tooth.

Optionally, determining a reference point coordinate based on a Euclidean distance between the first feature curve and a feature point coordinate of a tooth, the reference point coordinate being configured to adjust the dental characteristic parameters.

Optionally, determining a reference point coordinate in a vertical direction of a dental arch coordinate system of a tooth based on the first feature curve, the reference point coordinate being configured to adjust the dental characteristic parameters.

Optionally, determining a reference point coordinate based on the first feature curve and a first reference line, the first reference line being a reference line passing through a feature point of a tooth and extending along a direction of a first axis, the first axis being a coordinate axis of the dental arch coordinate system, and the first axis not being located in the second coordinate plane.

Optionally, a way of adjusting the dental characteristic parameters comprises at least one of: adjusting a position of a tooth in the first coordinate plane; adjusting a position of a tooth in the second coordinate plane; rotating a tooth; resolving a collision between adjacent teeth; and resolving a gap between adjacent teeth.

Optionally, a way of adjusting the dental characteristic parameters comprises at least one of: translating a position of a tooth in the first coordinate plane towards a direction closer to the first feature curve; translating a position of a tooth in the second coordinate plane towards a direction closer to the second feature curve; rotating a tooth around a first normal of the first coordinate plane as a rotation axis towards a first rotation direction, the first rotation direction being configured to make a long axis of the tooth in the first coordinate plane perpendicular to the first feature curve; rotating a tooth towards a second rotation direction, the second rotation direction being configured to make a first short axis parallel to a tangent of the second feature curve, the first short axis being a short axis of the tooth extending along a mesiodistal direction in the second coordinate plane; rotating a tooth towards a first inclination angle direction, the first inclination angle direction being a direction for reducing a difference between a long axis inclination angle of the tooth and a standard long axis inclination angle of the tooth, the long axis inclination angle being an angle between a long axis of the tooth and an extension direction of a first axis of the dental arch coordinate system; and rotating a tooth towards a second inclination angle direction based on a second rotation axis, the second inclination angle direction being a direction for reducing a difference between a long axis inclination angle and a standard long axis inclination angle, the second rotation axis passing through an intersection point of a second reference line and a long axis of the tooth, the second reference line being a reference line passing through a base point coordinate and parallel to a first axis, the base point coordinate being a base point coordinate on the second feature curve corresponding to a feature point coordinate of the tooth.

Optionally, the dental characteristic parameters comprise at least one of: an incisal ridge midpoint coordinate, a crown center coordinate, and a center of resistance coordinate of a tooth.

Optionally, the method for determining an orthodontic reference curve comprises: successively adjusting a dental characteristic parameter of a tooth to be adjusted with an adjustment step length, and updating the first feature curve accordingly.

Optionally, the method for determining an orthodontic reference curve comprises: successively adjusting a dental characteristic parameter of a tooth to be adjusted with an adjustment step length, and updating the second feature curve accordingly.

To achieve one of the objects of the application, an embodiment of the present application provides a method for designing a dental orthodontic appliance, comprising: obtaining an orthodontic reference curve based on a method for determining an orthodontic reference curve of any one of the technical solutions; and designing at least one set of dental orthodontic appliances based on a dental arch model data under a constraint of the orthodontic reference curve, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

Optionally, the method for designing a dental orthodontic appliance specifically comprises at least one of: obtaining a target point corresponding to a dental characteristic parameter of a tooth in the dental arch model on an orthodontic reference curve, obtaining a normal vector of a tangent at the target point on the orthodontic reference curve, determining an axial inclination amount based on the normal vector and a long axis of a corresponding tooth, and setting at least one orthodontic point corresponding to the tooth based on the axial inclination amount; or, obtaining a target point corresponding to a dental characteristic parameter of a tooth in the dental arch model on an orthodontic reference curve, obtaining and, with a first axis coordinate of the target point, constraining a setting range of an orthodontic point of a corresponding tooth in a first axis direction; or, obtaining a target point corresponding to a dental characteristic parameter of a tooth in the dental arch model on an orthodontic reference curve, obtaining a normal vector of a tangent at the target point on the orthodontic reference curve, and determining an occlusal depth parameter between the tooth and an opposing tooth along the normal vector to constrain a setting range of an orthodontic point of a corresponding tooth in a direction of the normal vector.

Optionally, obtaining a dental characteristic parameter of a dental arch model data; determining a third orthodontic reference curve in a second coordinate plane based on the dental characteristic parameter; and determining a target point corresponding to the dental characteristic parameter on the orthodontic reference curve based on a coordinate correspondence relationship between the third orthodontic reference curve and a second orthodontic reference curve.

To achieve one of the objects of the application, an embodiment of the present application provides a display method, comprising: displaying a Spee curve; the Spee curve is obtained based on a method for determining an orthodontic reference curve of any one of the technical solutions; and the Spee curve is used for designing an orthodontic plan.

A Spee curve, refers to a longitudinal occlusal curve of a mandibular dentition. In a lateral view, the Spee curve may refer to a smooth anteriorly convex arc starting from a buccal cusp of a second molar of a mandible or a maxilla, along buccal cusps of the mandibular dentition, passing through incisal edges of an incisor area, and may also refer to a line connecting incisal edges of mandibular incisors, cusps of canines, buccal cusps of premolars, mesial buccal cusps of molars, and distal buccal cusps of molars (i.e., a portion of the anteriorly convex arc located in the mandible).

To achieve one of the objects of the application, an embodiment of the present application provides a display method, comprising: displaying a dental arch model and a dental orthodontic appliance model; and the dental orthodontic appliance model is obtained based on a method for designing a dental orthodontic appliance of any one of the technical solutions.

To achieve one of the objects of the application, an embodiment of the present application provides a dental orthodontic appliance, wherein the dental orthodontic appliance is prepared based on an orthodontic reference curve determined by a method for determining an orthodontic reference curve of any one of the technical solutions, or prepared based on a method for designing a dental orthodontic appliance of any one of the technical solutions.

To achieve one of the objects of the application, an embodiment of the present application provides a computer storage medium, on which an application is stored, and when the application is executed, steps of a method for determining an orthodontic reference curve of any one of the technical solutions are implemented, or steps of a method for designing a dental orthodontic appliance of any one of the technical solutions are implemented.

To achieve one of the objects of the application, an embodiment of the present application provides an apparatus for determining an orthodontic reference curve, comprising: a first module, configured to obtain a dental characteristic parameter of a dental arch model data; a second module, configured to, based on the dental characteristic parameter, determine a first feature curve in a first coordinate plane and a second feature curve in a second coordinate plane, the first feature curve being a first orthodontic reference curve in a dental arch coordinate system, and the second feature curve being a dental arch curve; and a third module, configured to adjust the dental characteristic parameter, an adjusted dental characteristic parameter being configured to determine a second orthodontic reference curve, and the adjusted dental characteristic parameter adapting to a corresponding first feature curve and a corresponding second feature curve.

To achieve one of the objects of the application, an embodiment of the present application provides an apparatus for designing a dental orthodontic appliance, comprising: a fourth module, configured to obtain an orthodontic reference curve based on a method for determining an orthodontic reference curve of any one of the technical solutions; and a fifth module, configured to, under a constraint of the orthodontic reference curve, design at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

To achieve one of the objects of the application, an embodiment of the present application provides an electronic device, comprising a processor, a memory, and a communication bus, wherein the processor and the memory communicate with each other through the communication bus; the memory, configured to store an application; and the processor, configured to, when executing the application stored on the memory, implement steps of a method for determining an orthodontic reference curve of any one of the technical solutions, or implement steps of a method for designing a dental orthodontic appliance of any one of the technical solutions.

Compared with the prior art, the method for determining an orthodontic reference curve provided by the present application, after determining two feature curves for a dental arch model, adjusts a tooth to adapt to the feature curves to determine an orthodontic reference curve based on the adjusted tooth, which can automatically determine an orthodontic reference curve based on a given dental arch model and achieve customized configuration of the orthodontic reference curve based on a different dental arch model; providing two feature curves in two coordinate planes as a constraint enables the orthodontic reference curve determined by the method to simultaneously satisfy an actual situation in at least two dimensions, improving feasibility and accuracy, and saving unnecessary time consumption.

### BRIEF DESCRIPTION OF THE DRAWINGS

FIG.1 shows a schematic structural diagram of a dental orthodontic appliance in an embodiment of the present application.
FIG.2 shows a schematic structural diagram of an apparatus in an embodiment of the present application.
FIG.3 shows a schematic structural diagram of an electronic device in an embodiment of the present application.
FIG.4 shows a schematic structural diagram of a dental arch model from a first perspective in an embodiment of the present application.
FIG.5 shows a schematic diagram of steps of a method for determining an orthodontic reference curve in an embodiment of the present application.
FIG.6 shows a schematic diagram of a dental arch model from a second perspective when executing the method in an embodiment of the present application.
FIG.7 shows a schematic diagram of part of steps of an example of the method in an embodiment of the present application.
FIG.8 shows a schematic diagram of a dental arch model from a second perspective when determining a distance in an embodiment of the present application.
FIG.9 shows a schematic diagram of a dental arch model from a perspective when determining a distance in an embodiment of the present application.
FIG.10 shows a schematic diagram of a dental arch model from a second perspective when moving a tooth in an embodiment of the present application.
FIG. 11 shows a schematic diagram of a dental arch model from a second perspective when rotating a tooth in an embodiment of the present application.
FIG. 12 shows a schematic structural diagram of a first example of rotating tooth #26 in an embodiment of the present application.
FIG. 13 shows a schematic structural diagram of a second example of rotating tooth #26 in an embodiment of the present application.
FIG. 14 shows a schematic diagram of a part of teeth in a specific example of executing the method in an embodiment of the present application.
FIG. 15 shows a schematic diagram of steps of a method for designing a dental orthodontic appliance in an embodiment of the present application.
FIG. 16 shows a schematic diagram of part of steps of an example of the design method in an embodiment of the present application.
FIG. 17 shows a schematic diagram of a relationship between a feature point, an orthodontic point, and a target point in an embodiment of the present application.
FIG. 18 shows a schematic diagram of tooth #26 when executing an example of the design method in an embodiment of the present application.
FIG. 19 shows a schematic diagram of steps of a display method in an embodiment of the present application.
FIG. 20 shows a schematic diagram of steps of another display method in an embodiment of the present application.

### DETAILED DESCRIPTION

Specific embodiments of the present application will be described in detail below in conjunction with the accompanying drawings. However, these embodiments do not limit the present application. Structural, methodological, or functional transformations made by a person of ordinary skill in the art based on these embodiments are all included within a protection scope of the present application.

It should be noted that the term "comprising" or any other variation thereof is intended to cover a non-exclusive inclusion, such that a process, a method, an article, or a device that includes a series of elements includes not only those elements but also other elements not explicitly listed, or elements inherent to such process, method, article, or device. Furthermore, the terms "first", "second", "third", "fourth", etc. are used for descriptive purposes only and are not to be construed as indicating or implying relative importance. The terms "first", "second", "third", "fourth", etc. do not have a necessary correlation; for example, an embodiment provided in the present application that includes a "second" does not mean that the embodiment must include a "first", and so on.

A main concept of the present application is, after determining two feature curves corresponding to a dental arch model, to adjust a tooth through a mutual reference between the two feature curves, and to determine an orthodontic reference curve for the dental arch model based on the adjusted tooth; through a mutual constraint between the two feature curves, a process of adjusting the tooth and determining the orthodontic reference curve becomes more practical, achieving a customized and automated orthodontic reference determination for the dental arch model.

A feature curve and an orthodontic reference curve, may be a standard curve or an arc with a non-zero curvature at every position, may be a straight line or a polyline with a zero curvature at every position, and may also be a multi-segment spliced line with a non-zero curvature at a position and a zero curvature at another position.

A feature curve is used to represent a characteristic of a tooth in a dental arch model. The feature curve is particularly used to represent a relationship between a plurality of adjacent teeth.

An orthodontic reference curve is used to provide a reference for a dental orthodontic treatment. In an example, adjusting a tooth to move towards the orthodontic reference curve may achieve an orthodontic purpose or at least realize a staged orthodontic demand. A movement includes a rotation, a translation, or a combination of the translation and the rotation.

A mutual constraint between two feature curves includes adjusting a tooth with a first feature curve as a baseline, while referencing a second feature curve, so that the tooth at least does not deviate from a range specified by the second feature curve.

A mutual constraint between two feature curves includes adjusting a tooth with a second feature curve as a baseline, while referencing a first feature curve.

A mutual constraint between two feature curves includes adjusting a tooth with the two feature curves as a baseline simultaneously for a mutual reference.

The following will elaborate on a plurality of examples, technical principles, and corresponding technical effects of the present application in conjunction with the accompanying drawings.

An embodiment of the present application provides a dental orthodontic appliance.

In an example, a dental orthodontic appliance may be a bracket orthodontic appliance, including a bracket and an archwire. In some specific examples, the bracket orthodontic appliance includes a band, a buccal tube, a ligature wire, and a traction hook.

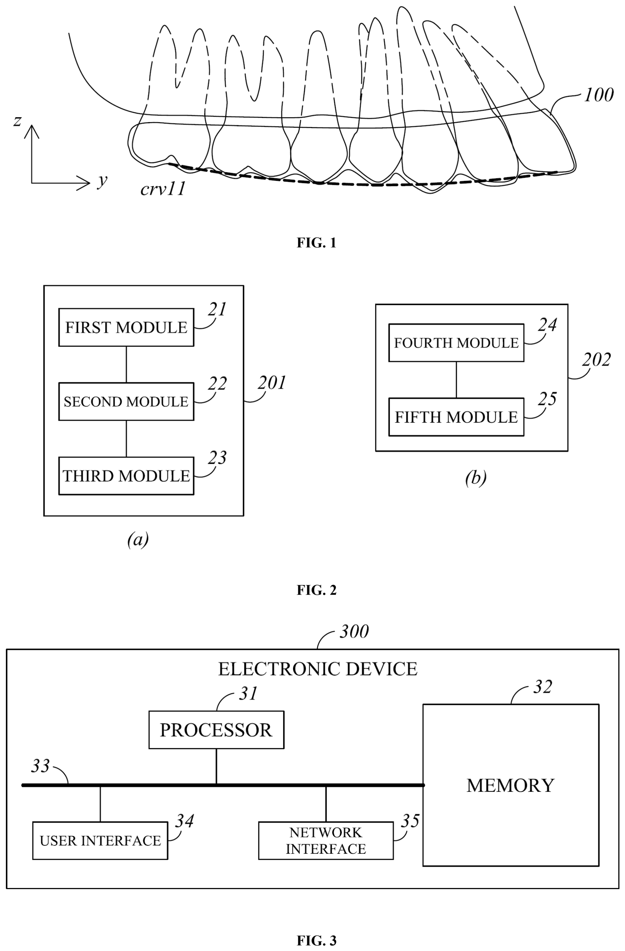

In an example, as shown in FIG. 1, a dental orthodontic appliance may be a shell-type orthodontic appliance, including an appliance body 100 forming a cavity for accommodating a tooth. In some specific embodiments, the shell-type orthodontic appliance includes an attachment fixed to a tooth or fixed to the appliance body.

A placement position of a dental orthodontic appliance may be inside an actual human oral cavity, or may be in a simulated physical oral model or a three-dimensional oral model.

A dental orthodontic appliance may be a physical device, or may be a three-dimensional model or a physical model of the dental orthodontic appliance.

In terms of a functional use, in some examples, a dental orthodontic appliance may also be a device used to implement an orthodontic treatment, or may be a retainer.

In an example, a dental orthodontic appliance is prepared based on an orthodontic reference curve.

The orthodontic reference curve is determined according to a method for determining an orthodontic reference curve. In an example, the method for determining an orthodontic reference curve may be any method provided in a subsequent embodiment.

In a specific example, the method for determining an orthodontic reference curve comprises at least one of the following steps:
obtaining a dental characteristic parameter of a dental arch model data;
based on the dental characteristic parameter, determining a first feature curve in a first coordinate plane and a second feature curve in a second coordinate plane, the first feature curve being a first orthodontic reference curve in a dental arch coordinate system; and
adjusting the dental characteristic parameter, an adjusted dental characteristic parameter being configured to determine a second orthodontic reference curve, and the adjusted dental characteristic parameter adapting to a corresponding first feature curve and a corresponding second feature curve.

In an example, after a second orthodontic reference curve crv11 is determined, a dental orthodontic appliance is designed so that an orthodontically treated tooth is arranged along the second orthodontic reference curve crv11.

In a specific example, as shown in FIG. 1, the second orthodontic reference curve crv11 is a Spee curve determined based on an adjusted dental characteristic parameter.

In an example, a dental orthodontic appliance is prepared based on a method for designing a dental orthodontic appliance.

The method for designing a dental orthodontic appliance may be any method provided in a subsequent embodiment.

In a specific example, the method for designing a dental orthodontic appliance comprises at least one of the following steps:
based on a method for determining an orthodontic reference curve, obtaining an orthodontic reference curve; and
under a constraint of the orthodontic reference curve, designing at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

In an example, after a second orthodontic reference curve crv11 is determined, an orthodontic point of a dental orthodontic appliance is located on a side of a feature point of a tooth closer to the second orthodontic reference curve crv11, so that a tooth treated by the dental orthodontic appliance may tend to conform to an arrangement of the second orthodontic reference curve crv11.

In a specific example, as shown in FIG. 1, the second orthodontic reference curve crv11 is a Spee curve determined based on an adjusted dental characteristic parameter.

In an embodiment of the present application, a computer storage medium is provided.

A computer storage medium may be set in a computer and may store an application. The computer storage medium may be any available medium from which the computer may access data, or may be a storage device such as a server or a data center that integrates one or more available media. The available medium may be a magnetic medium such as a floppy disk, a hard disk, or a magnetic tape, or an optical medium such as a DVD (Digital Video Disc), or a semiconductor medium such as an SSD (Solid State Disk).

In an example, when an application is executed, steps of a method for determining an orthodontic reference curve are implemented. In a specific example, the method for determining an orthodontic reference curve comprises at least one of the following steps:
obtaining a dental characteristic parameter of a dental arch model data;
based on the dental characteristic parameter, determining a first feature curve in a first coordinate plane and a second feature curve in a second coordinate plane, the first feature curve being a first orthodontic reference curve in a dental arch coordinate system; and
adjusting the dental characteristic parameter, an adjusted dental characteristic parameter being configured to determine a second orthodontic reference curve, and the adjusted dental characteristic parameter adapting to a corresponding first feature curve and a corresponding second feature curve.

A storage content of the computer storage medium may also be configured based on a method for determining an orthodontic reference curve in any technical solution provided below.

In an example, when an application is executed, steps of a method for designing a dental orthodontic appliance are implemented. In a specific example, the method for designing a dental orthodontic appliance comprises at least one of the following steps:
based on a method for determining an orthodontic reference curve, obtaining an orthodontic reference curve; and
under a constraint of the orthodontic reference curve, designing at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

A storage content of the computer storage medium may also be configured based on a method for designing a dental orthodontic appliance in any technical solution provided below.

As shown in (a) of FIG. 2, an embodiment of the present application provides an apparatus 201 for determining an orthodontic reference curve.

The apparatus 201 for determining an orthodontic reference curve comprises at least one of the following parts:
a first module 21, configured to obtain a dental characteristic parameter of a dental arch model data;
a second module 22, configured to, based on the dental characteristic parameter, determine a first feature curve in a first coordinate plane and a second feature curve in a second coordinate plane, the first feature curve being a first orthodontic reference curve in a dental arch coordinate system; and
a third module 23, configured to adjust the dental characteristic parameter, an adjusted dental characteristic parameter being configured to determine a second orthodontic reference curve, and the adjusted dental characteristic parameter adapting to a corresponding first feature curve and a corresponding second feature curve.

In an example, the second feature curve is a dental arch curve.

The apparatus 201 for determining an orthodontic reference curve may also be configured based on a method for determining an orthodontic reference curve in any technical solution provided below. Specifically, based on a correlation between steps, relevant steps may be implemented in a same module or different modules.

In an example, the first module 21, the second module 22, and the third module 23 may form an electrical connection or a communication connection to achieve a data transmission.

As shown in (b) of FIG. 2, an embodiment of the present application provides an apparatus 202 for designing a dental orthodontic appliance.

The apparatus 202 for designing a dental orthodontic appliance comprises at least one of the following parts:
a fourth module 24, configured to obtain an orthodontic reference curve based on a method for determining an orthodontic reference curve; and
a fifth module 25, configured to, under a constraint of the orthodontic reference curve, design at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

The apparatus 202 for designing a dental orthodontic appliance may also be configured based on a method for designing a dental orthodontic appliance in any technical solution provided below. Specifically, based on a correlation between steps, relevant steps may be implemented in a same module or different modules.

In an example, the fourth module 24 and the fifth module 25 may form an electrical connection or a communication connection to achieve a data transmission.

An apparatus or a module or a unit thereof may be specifically implemented by a computer chip or an entity, or by a product with a corresponding function. When describing the apparatus, although the apparatus is divided into a plurality of modules for separate description, in some examples, functions of the modules may be implemented in one or more same software or hardware.

An embodiment of the present application provides an electronic device 300, as shown in FIG. 3.

The electronic device 300 may be a computer, a mobile phone, a tablet computer, etc. The present application does not limit a specific type of the electronic device 300.

The electronic device 300 comprises at least one processor 31, at least one memory 32, and a communication bus 33.

The at least one processor 31 and the at least one memory 32 communicate with each other through the communication bus 33.

The memory 32 is configured to store an application.

In an example, the processor 31 is configured to, when executing the application stored on the memory 32, implement steps of a method for determining an orthodontic reference curve.

In a specific example, the method for determining an orthodontic reference curve comprises at least one of the following steps:
obtaining a dental characteristic parameter of a dental arch model data;
based on the dental characteristic parameter, determining a first feature curve in a first coordinate plane and a second feature curve in a second coordinate plane, the first feature curve being a first orthodontic reference curve in a dental arch coordinate system; and
adjusting the dental characteristic parameter, an adjusted dental characteristic parameter being configured to determine a second orthodontic reference curve, and the adjusted dental characteristic parameter is adapting to a corresponding first feature curve and a corresponding second feature curve.

The method for determining an orthodontic reference curve may also be configured based on any technical solution provided below.

In an example, the processor 31 is configured to, when executing the application stored in the memory 32, implement steps of a method for designing a dental orthodontic appliance.

In a specific example, the method for designing a dental orthodontic appliance comprises at least one of the following steps:
based on a method for determining an orthodontic reference curve, obtaining an orthodontic reference curve; and
under a constraint of the orthodontic reference curve, designing at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

The method for designing a dental orthodontic appliance may also be configured based on any technical solution provided below.

In an example, the communication bus 33 may comprise any number of buses and bridge circuits. In some examples, in addition to connecting the processor and the memory, the communication bus may also be used to connect a peripheral device or another peripheral circuit.

In an example, the electronic device 300 may comprise a user interface 34 and at least one network interface 35. The user interface 34 may comprise a display, a keyboard, a mouse, a trackball, a click wheel, a key, a button, a touchpad, or a touch screen, etc.

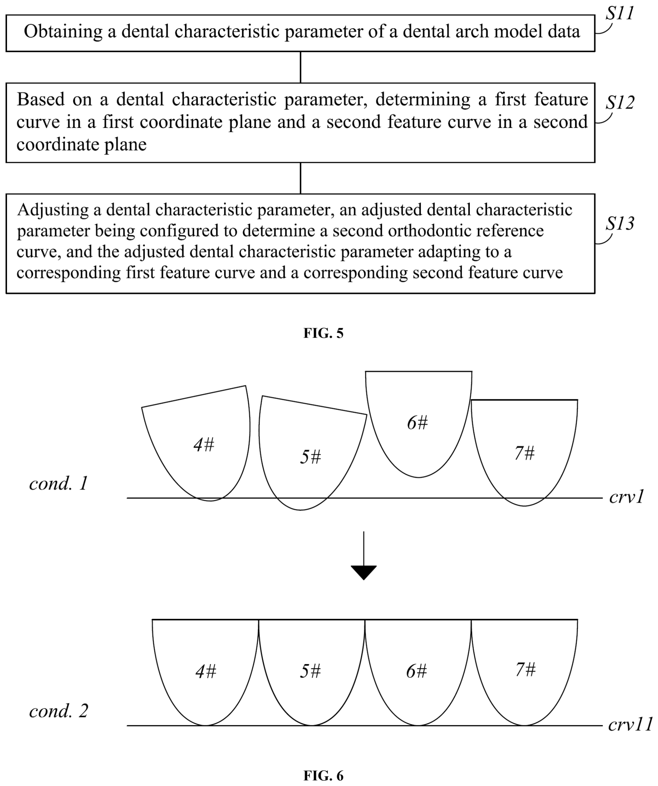

As shown in FIG. 5, an embodiment of the present application provides a method for determining an orthodontic reference curve.

An application or an instruction corresponding to the method may be loaded on an electronic device, an apparatus for determining an orthodontic reference curve, and/or a computer storage medium, may be loaded on a carrier of a display method provided below, may be loaded on a carrier for implementing a dental orthodontic appliance preparation, and may also be loaded in an apparatus for designing a dental orthodontic appliance, to achieve a corresponding technical effect.

The method for determining an orthodontic reference curve may specifically include the following steps.

Step S11, obtaining a dental characteristic parameter of a dental arch model data.

A dental arch model may be a model including teeth of all tooth positions, or may only include teeth of partial tooth positions. A dental arch model data may include information of all teeth in the dental arch model, or may only include information of partial teeth.

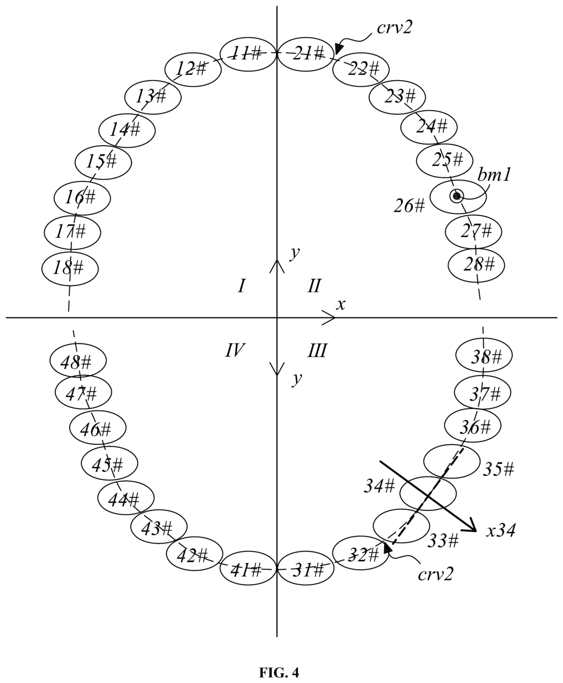

A dental characteristic parameter may be all characteristic parameters corresponding to all teeth in a dental arch model data, or may be a partial characteristic parameter corresponding to a partial tooth. In an example, a dental characteristic parameter includes a characteristic parameter of at least one tooth in a dental arch model data, for example, as shown in FIG. 4, including a characteristic parameter of tooth #26.

A dental characteristic parameter may be a parameter or a set thereof used to describe a characteristic such as a shape, a position, or a posture of a tooth. For example, the dental characteristic parameter may be a point cloud data corresponding to a crown shape, or a contour line corresponding to a crown outer contour. In an example, a dental characteristic parameter may be a feature point of a tooth or a coordinate thereof; in an example, a dental characteristic parameter may be a long axis of a tooth (an axis arranged in a growth direction) or a corresponding vector thereof, or a short axis of a tooth (an axis arranged in a mesiodistal direction) or a corresponding vector thereof, or an angle of the long axis relative to a certain coordinate axis, or an angle of the short axis relative to a certain coordinate axis.

In an example, a dental characteristic parameter includes an incisal ridge midpoint coordinate. For a posterior tooth, an incisal ridge midpoint coordinate may be a coordinate of a geometric center of an occlusal surface of the tooth.

In an example, a dental characteristic parameter includes a crown center coordinate. In some specific examples, a crown center coordinate may be a geometric center or a center of gravity of a space region occupied by a crown.

In an example, a dental characteristic parameter includes a center of resistance coordinate. When an external force acts on a tooth, a movement of the tooth is constrained by a surrounding bone tissue and a surrounding soft tissue, and all constraints are concentratedly expressed at one point or a small region, which is a center of resistance.

A dental characteristic parameter may include at least one of an incisal ridge midpoint coordinate, a crown center coordinate, and a center of resistance coordinate.

When a dental characteristic parameter is a coordinate value of a feature point on a tooth, a corresponding coordinate system may be a spatial coordinate system or a planar coordinate system.

A planar coordinate system may be a planar rectangular coordinate system or a polar coordinate system.

A spatial coordinate system may be a Cartesian coordinate system, or may be determined based on a dental arch model data.

A dental arch coordinate system determined based on a dental arch model data may include a first axis, a second axis, and a third axis. In an example, the first axis indicates an occlusal direction of an upper jaw and a lower jaw. In an example, the second axis indicates an anterior-posterior direction, and "anterior" and "posterior" are defined according to an anterior teeth area and a posterior teeth area. In an example, the third axis indicates a left-right direction.

In an example, a method for determining a dental arch coordinate system based on a dental arch model data includes at least one of the following steps:
step P1, obtaining a geometric center of a tooth in a dental arch model, and determining a first axis of a dental arch coordinate system and a first zero point thereof;
step P2, obtaining a first plane with the first axis as a normal thereof, and a geometric center of at least one pair of teeth, and determining a second axis of the dental arch coordinate system and a second zero point thereof; and
step P3, determining a third axis of the dental arch coordinate system based on the first axis and the second axis, obtaining a geometric center of at least one pair of teeth, and determining a third zero point.

For step P1, the first axis and the first zero point corresponding to the first axis can be determined through obtaining another feature point of a tooth.

The first zero point is an origin of the first axis.

In a specific example, step P1 may specifically include at least one of the following steps:
step P11, performing a principal component analysis on a geometric center of a tooth to determine a first axis; and
step P12, determining a first zero point based on a position of the geometric center of the tooth on the first axis.

Performing a principal component analysis (PCA) on a geometric center to reduce a dimension of a geometric center data, and selecting a required dimension according to a contribution to a data variation. The principal component analysis may specifically include: organizing a data into a matrix form, a data standardization, calculating a covariance matrix, calculating an eigenvalue and/or an eigenvector corresponding to the covariance matrix, selecting a principal component based on a magnitude of the eigenvalue (for example, selecting the eigenvector), and transforming a geometric center data based on the selected principal component.

For step P11, a specific example includes: determining a first axis based on a direction corresponding to a smallest eigenvalue.

For step P12, a specific example includes: obtaining a coordinate value of a geometric center of a tooth on a first axis, and determining a first zero point based on an average value of the coordinate value.

In an example, as shown in FIG. 1, a first axis is a z-axis.

For step P2, another feature point of a tooth may also be obtained to determine a second axis and a second zero point corresponding to the second axis.

The second zero point is an origin of the second axis.

In a specific example, step P2 may specifically include at least one of the following steps:
step P21, obtaining a position relationship of a geometric center of a pair of teeth in a first plane to determine a second axis; and
step P22, obtaining a position of the geometric center of the pair of teeth on the second axis to determine a second zero point.

In this way, a corresponding second axis may be determined based on a first axis, so as to construct a coordinate system matched with a dental arch model.

In a specific example, a pair of teeth refers to two teeth with corresponding numbers located in a same jaw. A number may be determined according to an FDI (Fédération Dentaire Internationale) tooth notation.

Taking FIG. 4 as an example, for an upper jaw, tooth #16 and tooth #26 may be defined to form a pair of teeth. For a lower jaw, tooth #46 and tooth #36 may be defined to form a pair of teeth.

For step P21, a dental arch model may include a plurality of pairs of teeth, two teeth in each pair of teeth have a position relationship, and a second axis may be determined based on a plurality of position relationships of the plurality of pairs of teeth.

For example, a geometric center of a tooth in each pair of teeth corresponds to two coordinate points in a first plane, a connecting line is formed by connecting the two coordinate points, and a plurality of connecting lines are formed by a plurality of pairs of teeth. A second axis may be determined based on the connecting line.

A specific example of step P21 includes: determining a perpendicular bisector of a connecting line of a pair of teeth at a first plane, and determining a second axis based on the perpendicular bisector.

A plurality of pairs of teeth may determine a plurality of perpendicular bisectors, and the plurality of perpendicular bisectors jointly determine a second axis. For example, the perpendicular bisector is normalized into a unit vector, and the second axis is obtained through an operation of the unit vector.

An operation mode of a unit vector may be to obtain an average vector of the unit vector. The average vector may be determined by an arithmetic mean calculation, or may be determined by a weighted mean calculation.

When a unit vector is calculated through a weighted mean, a posterior teeth area may be set to have a larger weight. For example, a unit vector of a corresponding pair of teeth from tooth #4 to tooth #8 (for example, tooth #14 to tooth #18, tooth #24 to tooth #28, tooth #34 to tooth #38, and tooth #44 to tooth #48) is set to have a larger weight.

Tooth #1 corresponds to include tooth #11, tooth #21, tooth #31, and tooth #41 in FIG. 4.

Tooth #2 corresponds to include tooth #12, tooth #22, tooth #32, and tooth #42 in FIG. 4.

Tooth #3 corresponds to include tooth #13, tooth #23, tooth #33, and tooth #43 in FIG. 4.

Tooth #4 (as shown in 4# in FIG. 6, FIG. 9, and FIG. 14) corresponds to include tooth #14, tooth #24, tooth #34, and tooth #44 in FIG. 4.

Tooth #5 (as shown in 5# in FIG. 6, FIG. 9, and FIG. 14) corresponds to include tooth #15, tooth #25, tooth #35, and tooth #45 in FIG. 4.

Tooth #6 (as shown in 6# in FIG. 6, FIG. 9, and FIG. 14) corresponds to include tooth #16, tooth #26, tooth #36, and tooth #46 in FIG. 4.

Tooth #7 (as shown in 7# in FIG. 6, FIG. 9, and FIG. 14) corresponds to include tooth #17, tooth #27, tooth #37, and tooth #47 in FIG. 4.

Tooth #8 corresponds to include tooth #18, tooth #28, tooth #38, and tooth #48 in FIG. 4.

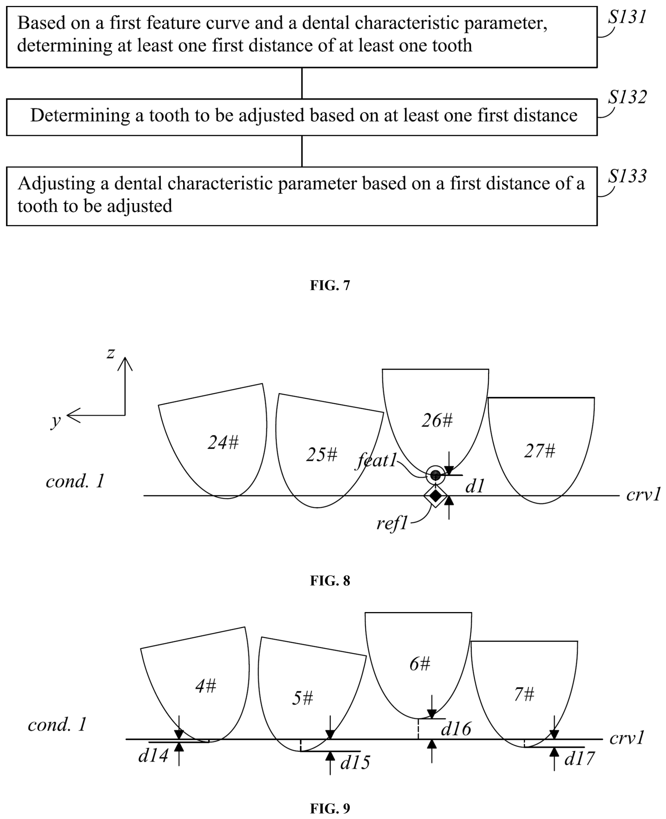

FIG. 8, FIG. 10, and FIG. 11 specifically show tooth #24, tooth #25, tooth #26, and tooth #27.

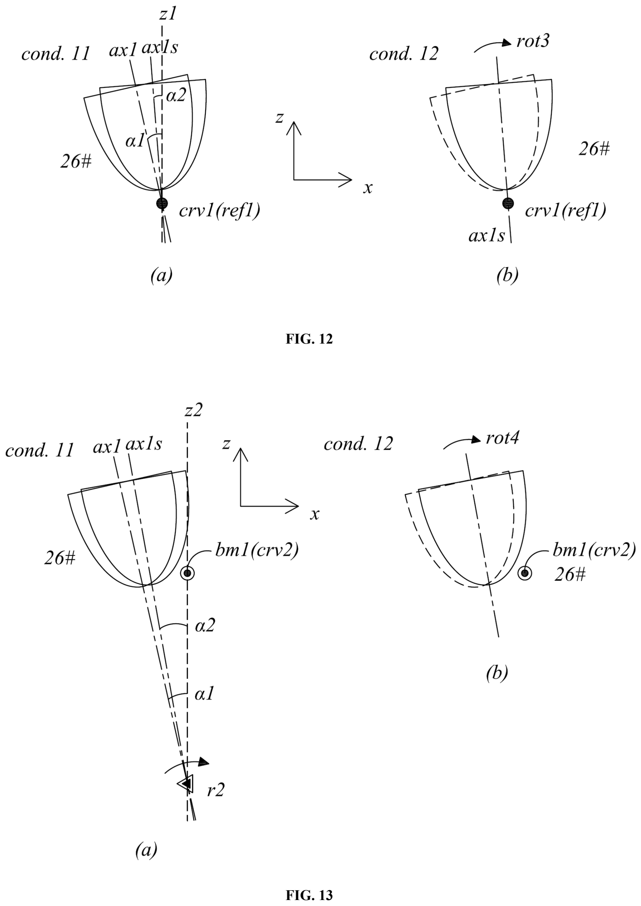

FIG. 12, FIG. 13, FIG. 17, and FIG. 18 specifically show tooth #26.

For step P22, a specific example includes: a second zero point may be determined through a plurality of pairs of teeth, or may be a specified single pair of teeth.

If a second zero point is determined based on a position of a plurality of pairs of teeth on a second axis, the second zero point may be determined by means of calculating an arithmetic mean, a weighted mean, etc. based on a coordinate of the plurality of pairs of teeth on the second axis.

If a second zero point is determined based on a position of a single pair of teeth on a second axis, a pair of teeth in a posterior teeth area may be specified, and an average value of coordinates of geometric centers of two teeth on the second axis is calculated to be used to determine the second zero point. For example, the second zero point is determined by a pair of teeth corresponding to tooth #6 (tooth #16 and tooth #26, or tooth #46 and tooth #36).

In an example, as shown in FIG. 1 and FIG. 4, a second axis is a y-axis.

A third zero point is an origin of a third axis.

In a specific example, step P3 may specifically include at least one of the following steps:
step P31, determining a third axis based on a vector corresponding to a first axis and a vector corresponding to a second axis; and
step P32, obtaining a position of a geometric center of a pair of teeth on the third axis to determine a third zero point.

In this way, a corresponding third axis may be determined based on a first axis and a second axis.

A specific example of step P31 includes: obtaining a cross product of a unit vector of a first axis and a unit vector of a second axis to determine a third axis.

In another specific example, a third axis may be not perpendicular to a plane formed by a first axis and a second axis (an angle between the third axis and the plane formed by the first axis and the second axis is not equal to 90 degrees), and at this time, the third axis may be determined through a corresponding degree between the third axis and the plane and a unit vector of two axes.

In another specific example, a third axis may also be determined through a connecting line of a pair of teeth in a same jaw. For example, a unit vector of a connecting line of a plurality of pairs of teeth in a same jaw is determined, and an average vector of these unit vectors is calculated to determine the third axis.

A specific example of step P32 includes: a third zero point may be determined through a plurality of pairs of teeth, or may be a specified single pair of teeth.

If a third zero point is determined based on a position of a single pair of teeth on a third axis, a specific pair of teeth may be specified, and an average value of coordinates of geometric centers of two teeth on a coordinate axis of the third axis is calculated to be used as the third zero point.

If a third zero point is determined based on a position of a plurality of pairs of teeth on a third axis, the third zero point may be determined by means of calculating an arithmetic mean, a weighted mean, etc. based on a coordinate of a midpoint of a connecting line of the plurality of pairs of teeth on the third axis. For example, the plurality of pairs of teeth may be all pairs of teeth in a dental arch model.

In an example, as shown in FIG. 4, a third axis is an x-axis.

In an example, an origin coordinate of a dental arch coordinate system is determined based on a first zero point, a second zero point, and a third zero point. When the first zero point corresponds to a z-axis, the second zero point corresponds to a y-axis, and the third zero point corresponds to an x-axis, the origin coordinate is in a form of: (the third zero point, the second zero point, the first zero point).

Step S12, based on a dental characteristic parameter, determining a first feature curve in a first coordinate plane and a second feature curve in a second coordinate plane.

The first feature curve is located in the first coordinate plane and the second feature curve is located in the second coordinate plane. That is intended to express that the two feature curves are dental characteristics corresponding to different orientation dimensions, and does not limit that the two feature curves must be formed by mapping the dental characteristic parameter to a coordinate plane.

If a first coordinate plane defines a first orientation dimension, a first feature curve correspondingly represents a position, a posture, or a shape characteristic of a dental characteristic parameter in the first orientation dimension. In an example, the first feature curve may represent a distribution of a plurality of teeth in the first orientation dimension.

If a second coordinate plane defines a second orientation dimension, a second feature curve correspondingly represents a position, a posture, or a shape characteristic of a dental characteristic parameter in the second orientation dimension. In an example, the second feature curve may represent a distribution of a plurality of teeth in the second orientation dimension.

In an example, a first coordinate plane and a second coordinate plane are two intersecting planes in a spatial coordinate system. The spatial coordinate system is a Cartesian coordinate system or a dental arch coordinate system.

In a specific example, a first coordinate plane and a second coordinate plane are perpendicular to each other.

In an example, a first feature curve is determined by fitting a dental characteristic parameter.

In a specific example, a first feature curve is determined by fitting a dental characteristic parameter processed by a B-spline function. In some other specific examples, the first feature curve may also be determined by fitting a dental characteristic parameter processed by a cubic spline function.

In an example where a B-spline function is implemented to fit a first feature curve, the present application specifically includes steps of: determining a feature point of a tooth, determining a B-spline basis function (which may include: at least one of determining a curve degree and calculating a knot vector), and determining a B-spline curve (which may include: at least one of calculating a B-spline basis function value, determining a curve point, determining a parameter value range, and performing a point-by-point control or adjustment).

In an example, a second feature curve is determined by fitting a dental characteristic parameter.

In a specific example, a second feature curve is determined by fitting a dental characteristic parameter processed by a fourth-order polynomial.

In an example where a fourth-order polynomial is implemented to fit a second feature curve, the present application includes steps of: determining feature points of teeth, respectively determining a fourth-order polynomial (in a form of: *y = ax*⁴ + *bx*³ *+ cx*² *+ dx + e* ) based on coordinates of the feature points in a second coordinate plane, solving coefficients of the fourth-order polynomial to fix the fourth-order polynomial, and determining a corresponding curve based on the fourth-order polynomial.

In a specific example, a second feature curve is determined by fitting a parameter set determined by constructing an ellipse for a feature point corresponding to a dental characteristic parameter.

In an example where an ellipse is implemented to fit a second feature curve, the present application includes steps of: determining a feature points of teeth, respectively determining an ellipse equation (in a form of: *Ax*² *+ Bxy + Cy*² *+ Dx + Ey + F* = 0) for coordinates of the feature points in a second coordinate plane, solving coefficients of the ellipse equation to fix the ellipse equation, and determining a corresponding elliptical curve under an ellipse constraint condition (for example, *B*² - 4*AC* <0).

In an example, a first feature curve is a first orthodontic reference curve in a dental arch coordinate system.

Defining a first feature curve as an orthodontic reference curve is intended to express that the first feature curve may be any curve used for an orthodontic treatment. For example, the first feature curve may be a Steiner Line, an auxiliary line related to a Downs Analysis (for example, an AB line), an auxiliary line related to a Ricketts Analysis (for example, an E line), etc.

A first orthodontic reference curve and a second orthodontic reference curve mentioned below may refer to different types of curves. At this time, two types of orthodontic reference curves may be applied simultaneously to provide an auxiliary reference for an orthodontic process. Since the first orthodontic reference curve is determined before a tooth adjustment, a manner in which the two types of orthodontic reference curves provide an orthodontic assistance may be that the two types of orthodontic reference curves function separately and in parallel, or may be that the two types of orthodontic reference curves function in comparison with each other and jointly.

A first orthodontic reference curve and a second orthodontic reference curve mentioned below may refer to a same type of curve. At this time, the second orthodontic reference curve may be a curve that is determined after being updated and may be applied to provide an auxiliary function in an orthodontic process, and the first orthodontic reference curve corresponds to a curve that is before being updated and cannot be directly applied for an orthodontic assistance.

In an example, a first orthodontic reference curve may refer to one type of curve; in another example, the first orthodontic reference curve may refer to a combination of a plurality of types of curves, and at this time, a first feature curve is one of the first orthodontic reference curves.

In an example, a second orthodontic reference curve may refer to one type of curve; in another example, the second orthodontic reference curve may refer to a combination of a plurality of types of curves.

In a specific example, a first orthodontic reference curve is a Spee curve.

In a specific example, a first feature curve is a Spee curve.

The Spee curve is a curve proposed by a German anatomist Ferdinand Graf von Spee in the 1890s. By evaluating and adjusting the Spee curve to be close to an ideal state, an occlusal relationship and an aesthetic of a patient may be improved, a correct coordination between an upper dental arch and a lower dental arch is promoted, and a chewing efficiency and a coordinated movement of a maxillofacial muscle are facilitated.

In an example, a Spee curve is a smooth anteriorly convex arc formed in a lateral view, starting from a buccal cusp of a second molar of a mandible or a maxilla, along buccal cusps of a mandibular dentition, and passing through incisal edges of an incisor area.

In an example, as shown in FIG. 1, a lateral view refers to a yz coordinate plane in a Cartesian coordinate system or a dental arch coordinate system. In an example where a first feature curve is a Spee curve, a first coordinate plane includes the yz coordinate plane.

A second orthodontic reference curve crv11 shown in FIG. 1 may be determined after a first orthodontic reference curve is adjusted. The second orthodontic reference curve crv11 in FIG. 1 takes a portion of a complete Spee curve located in an upper jaw.

A first feature curve crvl and a second orthodontic reference curve crv11 shown in FIG. 6, FIG. 8, FIG. 9,

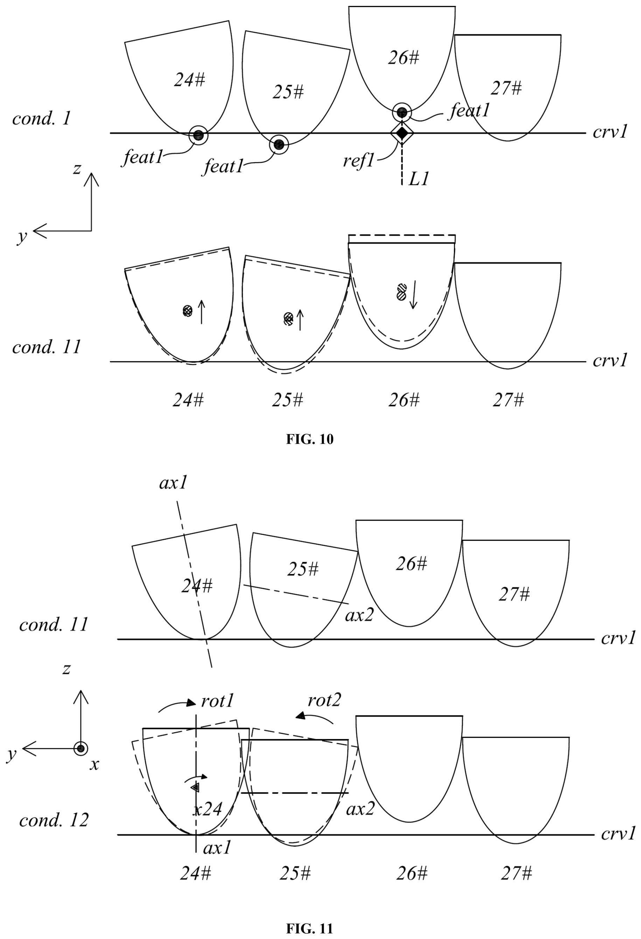

FIG. 10, FIG. 11, FIG. 14, FIG. 17, and FIG. 18 are examples formed after a Spee curve is deformed into a straight line extending along a y direction. The first feature curve crv1 and the second orthodontic reference curve crv11 in these figures take a portion of a complete Spee curve located in a posterior teeth area.

In a specific example, a first feature curve is a Spee curve and is determined through a B-spline function fitting. In this way, a smooth first feature curve that does not strictly pass through a feature point corresponding to each dental characteristic parameter may be obtained, which facilitates adjusting the dental characteristic parameter accordingly.

In an example, a second feature curve is a dental arch curve.

A dental arch curve refers to a visual contour line of a tooth crown in an oral cavity, and presents an elliptical or parabolic shape in a horizontal view.

Referring to FIG. 4, a second feature curve crv2 in a quadrant I and a quadrant II is a dental arch curve corresponding to an upper jaw (or maxilla), and a second feature curve crv2 in a quadrant IV and a quadrant III is a dental arch curve corresponding to a lower jaw (or mandible). FIG. 4 is only an example; generally, a dental arch curve of an upper jaw is wider than a dental arch curve of a lower jaw.

In a specific example, a second feature curve is a dental arch curve and is determined through a fourth-order polynomial fitting or an ellipse fitting.

Step S13, adjusting a dental characteristic parameter, an adjusted dental characteristic parameter being configured to determine a second orthodontic reference curve, and the adjusted dental characteristic parameter adapting to a corresponding first feature curve and a corresponding second feature curve.

Adjusting a dental characteristic parameter includes adjusting at least one of a shape, a position, and a posture of a tooth.

In an example, a second orthodontic reference curve may be determined by using a dental characteristic parameter, and the second orthodontic reference curve is used to assist an orthodontic treatment. At this time, a process of adjusting the dental characteristic parameter may correspond to an actual orthodontic operation.

For example, when a determination process of a second orthodontic reference curve includes an adjustment of a tooth shape, the tooth shape may also be adjusted during an orthodontic process. An adjustment mode of a tooth shape includes at least one of: a tooth cutting (for example, a proximal enamel reduction), a tooth reshaping, an addition of a resin material, a dental brace, and a crown.

For example, when a determination process of a second orthodontic reference curve includes an adjustment of a tooth position, a dental orthodontic appliance may be configured during an orthodontic process to apply a corresponding acting force to a tooth to promote such a position movement.

For example, when a determination process of a second orthodontic reference curve includes an adjustment of a tooth posture, a dental orthodontic appliance may be configured during an orthodontic process to apply a corresponding rotating force to a tooth to adjust a torsion and a torque of the tooth.

An adjusted dental characteristic parameter is used for determining a second orthodontic reference curve. Based on this, an output of a method for determining an orthodontic reference curve provided by the present application may be the second orthodontic reference curve itself, or may be the dental characteristic parameter used for determining the second orthodontic reference curve.

In an example, a dental characteristic parameter may be adjusted based on a second feature curve, and a first orthodontic reference curve is updated to form a second orthodontic reference curve.

In a specific example, a first orthodontic reference curve is a first feature curve. For example, the first orthodontic reference curve is an initial Spee curve; a second orthodontic reference curve includes a Spee curve that may participate in an orthodontic treatment.

For example, a dental characteristic parameter is adjusted based on a dental arch curve, and an initial Spee curve as a first feature curve is updated to form a Spee curve that may participate in an orthodontic treatment.

In a specific example, a first orthodontic reference curve includes a first feature curve and a second feature curve. For example, the first orthodontic reference curve includes an initial Spee curve and an initial dental arch curve; a second orthodontic reference curve includes a Spee curve and/or a dental arch curve that may participate in an orthodontic treatment.

For example, a dental characteristic parameter is adjusted based on a dental arch curve, and an initial Spee curve as a first feature curve and an initial dental arch curve as a second feature curve are updated to form a Spee curve and/or a dental arch curve that may participate in an orthodontic treatment.

In an example, a dental characteristic parameter may be adjusted based on a first feature curve and a second feature curve, and a first orthodontic reference curve is updated to form a second orthodontic reference curve.

In a specific example, a first orthodontic reference curve is a first feature curve. For example, the first orthodontic reference curve is an initial Spee curve; a second orthodontic reference curve includes a Spee curve that may participate in an orthodontic treatment.

For example, a dental characteristic parameter is adjusted based on a dental arch curve and a Spee curve, and an initial Spee curve as a first feature curve is updated to form a Spee curve that may participate in an orthodontic treatment.

In a specific example, a first orthodontic reference curve includes a first feature curve and a second feature curve. For example, the first orthodontic reference curve includes an initial Spee curve and an initial dental arch curve; a second orthodontic reference curve includes a Spee curve and/or a dental arch curve that may participate in an orthodontic treatment.

For example, a dental characteristic parameter is adjusted based on a dental arch curve and a Spee curve, and an initial Spee curve as a first feature curve and an initial dental arch curve as a second feature curve are updated to form a Spee curve and/or a dental arch curve that may participate in an orthodontic treatment.

An adjusted dental characteristic parameter is adapted to a corresponding first feature curve and a corresponding second feature curve. Based on this, whether the dental characteristic parameter is adapted to a corresponding first feature curve and a corresponding second feature curve can be used as a criterion to determine whether an adjustment of the dental characteristic parameter is completely adjusted.

When a dental characteristic parameter is adjusted, a first feature curve and/or a second feature curve may be redetermined as the dental characteristic parameter changes.

In an example, a first feature curve is refitted as a dental characteristic parameter changes. What is adapted to an adjusted dental characteristic parameter may be an updated first feature curve.

When a dental characteristic parameter is adjusted, a first feature curve and/or a second feature curve may remain relatively unchanged.

In an example, during a process of adjusting a dental characteristic parameter, a second feature curve remains relatively unchanged. What is adapted to an adjusted dental characteristic parameter may be an initially determined second feature curve.

In an example, an adaptation between a dental characteristic parameter and a feature curve may be that a feature point corresponding to the dental characteristic parameter is located on a first feature curve, a feature point corresponding to the dental characteristic parameter is located on a second feature curve, or a distance between a feature point corresponding to the dental characteristic parameter and a first feature curve is within an allowable error range, or a distance between a feature point corresponding to the dental characteristic parameter and a second feature curve is within an allowable error range.

In another example, whether a dental characteristic parameter is adapted to a feature curve may also be determined by judging whether the dental characteristic parameter satisfies a functional relationship corresponding to the feature curve.

Through implementing steps S11 to S13, a dental characteristic parameter may be adjusted with reference to a first feature curve and a second feature curve to update and obtain a second orthodontic reference curve. Since the two feature curves and the dental characteristic parameter are both determined based on a dental arch model data, and an orthodontic reference curve may be determined based on a tooth adjustment, an obtained orthodontic reference is more capable of adapting to an actual situation of a dental arch model; since an adjustment process of the dental characteristic parameter is performed under a mutual constraint of two feature curves in two dimensions, even if the adjustment process is automatically generated, the adjustment process does not deviate from reality, and has a higher accuracy and efficiency.

In combination with FIG. 6, taking an arrangement adjustment of tooth #4 to tooth #7 as an example, through executing steps S11 to S13, a first feature curve crv1 in a first condition cond. 1, after an adjustment of a dental characteristic parameter and a corresponding tooth, may determine a second orthodontic reference curve crv11 in a second condition cond. 2.

Under a guidance of the second orthodontic reference curve erv11, corresponding teeth may form an aesthetic and healthy arrangement. Since an adjustment process takes a consideration of a second feature curve crv2 shown in FIG. 4 into account, a good orthodontic auxiliary effect may be achieved in at least two dimensions.

In an example provided by the present application, a method for determining an orthodontic reference curve includes at least one of the following steps:

Step S131, based on a first feature curve and a dental characteristic parameter, determining at least one first distance of at least one tooth;
the first distance being a distance of the at least one tooth relative to the first feature curve.

As shown in FIG. 8, for example, under a first condition cond. 1, tooth #24 to tooth #27 are arranged as shown. Based on a dental characteristic parameter, a first feature curve crv1 may be determined. At least tooth #26 in a dental arch model has a first distance d1 relative to the first feature curve crv1.

Step S132, determining a tooth to be adjusted based on at least one first distance.

In an example, determining whether a tooth is a tooth to be adjusted based on a first distance of the tooth.

In a specific example, based on a numerical relationship between a first distance of a tooth and a preset distance, determining a tooth with the first distance greater than or less than the preset distance as a tooth to be adjusted.

In a specific example, based on a relationship between a first distance of a tooth and a first distance of another tooth, determining a tooth with a larger difference as a tooth to be adjusted, or determining a tooth with a smaller difference as a tooth to be adjusted, or determining a tooth with a larger first distance as a tooth to be adjusted, or determining a tooth with a smaller first distance as a tooth to be adjusted.

In another example, all teeth in a dental arch model may be determined as teeth to be adjusted, regardless of whether a first distance of the teeth is formed.

Step S133, adjusting a dental characteristic parameter based on a first distance of a tooth to be adjusted.

In an example, when a first distance d1 of a tooth is larger than a preset distance or a first distance of another tooth, a dental characteristic parameter may be adjusted to reduce the first distance d1 of the tooth, so as to adjust the tooth towards a trend of adapting to a first feature curve crv1.

In an example, when a difference between a first distance d1 of a tooth and a first distance of another tooth is large, a dental characteristic parameter may be adjusted to make the first distance d1 of the tooth tend to be close to the first distance of another tooth, so as to form a mutually adapted dental characteristic parameter and a first feature curve. At this time, an adjustment of the dental characteristic parameter may cause a first distance to become larger, or may cause the first distance to become smaller.

In an example, steps S131 to S133 are executed in sequence.

In an example, steps S131 to S133 are included in step S13.

In an example provided by the present application, a method for determining an orthodontic reference curve includes at least one of the following steps:

Step S1311, based on a first feature curve and a dental characteristic parameter, determining a plurality of first distances of a plurality of teeth relative to the first feature curve;
the first distance being a distance of a tooth relative to the first feature curve.

The plurality of teeth may be two or more teeth. The plurality of teeth correspond to form two or more first distances relative to a first feature curve.

As shown in FIG. 9, for example, under a first condition cond. 1, tooth #4 to tooth #7 are arranged as shown. Based on a dental characteristic parameter, a first feature curve crv1 may be determined. Based on the dental characteristic parameter and the first feature curve crv1, a first distance d14 of tooth #4, a first distance d15 of tooth #5, a first distance d16 of tooth #6, and a first distance d17 of tooth #7 may be determined.

Tooth #4 to tooth #7 may correspond to tooth #14 to tooth #17, tooth #24 to tooth #27, tooth #34 to tooth #37, or tooth #44 to tooth #47 in FIG. 4.

Step S1321, based on a dispersion degree among a plurality of first distances, determining a tooth satisfying a dispersion threshold condition as a tooth to be adjusted.

In this way, a first distance significantly inconsistent with another first distance and a corresponding tooth thereof may be determined, and the tooth is used as a tooth to be adjusted, and is adjusted to tend to adapt to a first feature curve, so as to determine a second orthodontic reference curve.

In an example, a dispersion threshold is determined based on dispersion degree of a plurality of first distances; the dispersion threshold defines a threshold interval, and a tooth with a first distance falling into the threshold interval is determined as a tooth to be adjusted by comparing a numerical relationship between the first distance and the dispersion threshold.

In a specific example, a dispersion threshold (for example, a mean ± 2 × a standard deviation) may be determined based on a central tendency value (for example, a mean or a median) and a dispersion value (for example, a standard deviation).

For example, in an example disclosed in FIG. 9, when it is determined that a first distance d16 of tooth #6 does not fall into a dispersion threshold interval, at least tooth #6 may be determined as a tooth to be adjusted; the dispersion threshold interval is determined based on a first distance of tooth #4 to tooth #7.

In a specific example, after an adjustment of a dental characteristic information of tooth #6 is completed, a new first feature curve may be determined based on an adjusted tooth #4 to an adjusted tooth #7, and then a new first distance and a new dispersion threshold are calculated for a new round of adjustment.

Step S1322, based on sequence information of a plurality of first distances, determining a tooth to be adjusted.

In this way, a tooth ranking at a front or a back may be determined as a tooth to be adjusted based on an ordering of a first distance. In an example, a sequence of a tooth adjustment may also be determined based on the ordering of the first distance.

In an example, sequence information is determined by sorting first distances according to numerical values of the first distances.

In an example, sequence information is determined by arranging first distances in a descending order, and a tooth corresponding to a first distance ranking at a front is determined as a tooth to be adjusted. In this way, an offset of a tooth relative to a first feature curve may be reduced in a targeted manner.

For example, in an example disclosed in FIG. 9, a descending order of first distances is: tooth #6, tooth #5, tooth #7, and tooth #4. Tooth #6 ranking first in sequence information may be determined as a tooth to be adjusted; or tooth #6 and tooth #5 respectively ranking first and second in the sequence information may be determined as teeth to be adjusted.

In a specific example, after an adjustment of a dental characteristic information is completed, a new first feature curve may be determined based on an adjusted tooth #4 to an adjusted tooth #7, and then a new first distance and a new sequence information are calculated for a new round of adjustment.

In an example, step S1311 and step S1321 are executed in sequence.

In an example, step S1311 and step S1322 are executed in sequence.

In an example, step S1311 is included in step S131 or step S13.

In an example, step S1321 is included in step S132 or step S13.

In an example, step S1322 is included in step S132 or step S13.

In an example provided by the present application, a method for determining an orthodontic reference curve includes a step: based on feature point coordinate information and reference point coordinate information, determining at least one first distance of at least one tooth.

In a specific example, a first distance is used for determining a tooth to be adjusted. In a specific example, the first distance is used for adjusting a dental characteristic parameter.

As shown in FIG. 8, in an example, a first distance d1 is determined based on a feature point feat1 and a reference point refl.

Feature point coordinate information is configured to indicate a feature point coordinate of at least one tooth. In an example where a tooth includes tooth #26, a feature point coordinate correspondingly includes a coordinate of a feature point feat1.

Reference point coordinate information is configured to indicate a reference point coordinate on a first feature curve corresponding to a feature point coordinate of at least one tooth. In an example where a tooth includes tooth #26, a reference point coordinate correspondingly includes a coordinate of a reference point refl.

In another example, a first distance may also be determined through an occlusal surface distribution information of a tooth or an incisal ridge distribution information of a tooth and a first feature curve, and a distance from a feature point coordinate information to the first feature curve may also be directly calculated, and a minimum value in distance values is selected as the first distance.

In a specific example, determining a first distance based on feature point coordinate information and reference point coordinate information may include: determining a position relationship between a feature point feat1 and a point on a first feature curve crvl, determining a point satisfying a preset condition in the position relationship as a reference point refl, and fixing a reference point coordinate information.

In a specific example, determining a first distance based on feature point coordinate information and reference point coordinate information may include: determining a point on a first feature curve crv1 located in a first direction of a feature point feat1 as a reference point refl, and fixing a reference point coordinate information.

In a specific example, a first distance d1 may be a distance between a feature point feat1 and a reference point refl. In a specific example, the first distance d1 may be determined by calculating a Euclidean distance between a feature point coordinate information and a reference point coordinate information.

An example discloses a technical solution for determining a first distance based on reference point coordinate information. In some examples, the reference point coordinate information may be determined based on the first distance.

In an example provided by the present application, a method for determining an orthodontic reference curve includes a step: based on a Euclidean distance between a first feature curve and a feature point coordinate of a tooth, determining a reference point coordinate.

In a specific example, a reference point corresponding to a feature point on a first feature curve may be determined based on a Euclidean distance. Specifically, a Euclidean distance from a feature point of a tooth to the first feature curve may be a distance between the feature point and a point on the first feature curve having a shortest distance from the feature point. When the Euclidean distance is determined, a point on the first feature curve having the shortest distance from the feature point of the tooth may be determined as a reference point, so as to determine a reference point coordinate.

In a specific example, a Euclidean distance between a feature point of a tooth and a plurality of points on a first feature curve may be calculated first, and a feature point with a shortest distance is determined as a reference point, so as to determine a reference point coordinate.

In a specific example, a reference point coordinate is configured to adjust a dental characteristic parameter.

In a specific example, a dental characteristic parameter may be adjusted to cause a whole tooth or a feature point of the tooth to move towards a direction closer to a reference point. A movement of the tooth of feature point includes a translation, a rotation, or a deformation.

In an example provided by the present application, a method for determining an orthodontic reference curve includes a step: based on a first feature curve, determining a reference point coordinate in a vertical direction of a dental arch coordinate system of a tooth.

In a specific example, a vertical direction may be a growth direction of a tooth. In a specific example, a vertical direction may be a direction perpendicular to an occlusal surface of a tooth. In a specific example, a vertical direction may be a long axis direction of a tooth. In a specific example, a vertical direction may be a vertical direction of a Cartesian coordinate system.

In an example shown in FIG. 8 and FIG. 10, a vertical direction is a direction of a first axis z of a dental arch coordinate system. A reference point determined in this way, when being used for determining a first distance, may cause the determined first distance to be close to or even equal to a vertical distance from a feature point of a tooth to a first feature curve crvl, which may improve an efficiency of a tooth adjustment process and a second orthodontic reference curve determination process. When being used for adjusting a dental characteristic parameter, a direction of a tooth movement may also be determined while a reference point is determined, which may accelerate a process of adjusting the dental characteristic parameter.

In an example, a vertical direction may also be a direction passing through a feature point of a tooth and perpendicular to a first feature curve. A tangent point formed by a corresponding tangent and the first feature curve may be a reference point.

In a specific example, a reference point coordinate is configured to adjust a dental characteristic parameter.

In a specific example, a dental characteristic parameter may be adjusted to cause a whole tooth or a feature point of the tooth to move towards a direction closer to a reference point. A movement of the tooth of feature point includes a translation, a rotation, or a deformation.

In an example, a method for determining an orthodontic reference curve includes a step: based on a first feature curve and a first reference line, determining a reference point coordinate.

A first reference line is a reference line passing through a feature point of a tooth and extending along a direction of a first axis.

A first axis is a coordinate axis of a dental arch coordinate system, and the first axis is not located in a second coordinate plane.

Referring to FIG. 10, for example, for tooth #26, if a second coordinate plane is defined by a second axis y and a third axis x, a first axis z of a dental arch coordinate system is not located in the second coordinate plane. In a specific example, the first axis z is perpendicular to the second coordinate plane.

A first reference line L1 passing through a feature point feat1 of tooth #26 and extending along a direction of a first axis z is determined. Based on this, a reference point refl may be determined based on the first reference line L1 and a first feature curve crv1.

In a specific example, a reference point refl is an intersection point of a first reference line L1 and a first feature curve crv1.

In an example provided by the present application, a way of adjusting a dental characteristic parameter includes: adjusting a position of a tooth in a first coordinate plane.

In a specific example, a first coordinate plane includes a yz plane constructed based on a first axis z and a second axis y, as shown in FIG. 10.

For example, during a process from a first condition cond. 1 to a first intermediate condition cond. 11, a position of tooth #24, tooth #25, and tooth #26 in a first coordinate plane may be adjusted, so that the teeth respectively move towards a direction adapted to a first feature curve crv1.

In FIG. 10, a dashed line part shows a position of each tooth and a geometric center of a crown thereof before an adjustment (i.e., at a first condition cond. 1), and a solid line part shows the position of each tooth and the geometric center of the crown thereof after the adjustment (i.e., at a first intermediate condition cond. 11). It can be seen that a direction and an amount of a position adjustment of each tooth in a first coordinate plane may be the same or different.

In an example provided by the present application, a way of adjusting a dental characteristic parameter includes: translating a position of a tooth in a first coordinate plane towards a direction closer to a first feature curve.

In a specific example, a direction closer to a first feature curve may refer to a direction in which a feature point of a tooth approaches the first feature curve.

As shown in FIG. 10, although during a process from a first condition cond. 1 to a first intermediate condition cond. 11, tooth #24 and tooth #25 move upward (towards a positive direction of a first axis z), since at the first condition cond. 1, a feature point feat1 of each of the two teeth is located below a first feature curve crv1 (on a negative direction side of the first axis z), an upward movement of the two teeth substantially shortens a distance between the feature point feat1 and the first feature curve crv1.

In an example, after a type of adjustment is completed, a first feature curve crv1 is redetermined based on an adjusted dental characteristic parameter. For example, at a first intermediate condition cond. 11, since an adjustment of a tooth position in a first coordinate plane is completed, the first feature curve crv1 is updated.

In an example, after a set of adjustments is completed, a first feature curve crv1 is then redetermined based on an adjusted dental characteristic parameter. For example, in combination with FIG. 10 and FIG. 11, during a process from a first intermediate condition cond. 11 to a second intermediate condition cond. 12, an adjustment of a tooth rotation is completed. If a set of adjustments is defined as completing one position adjustment in a first coordinate plane and one tooth rotation, a rotation adjustment shown in FIG. 11 is still performed based on an original first feature curve crv1, until the first feature curve crv1 is updated when the adjustment of the tooth rotation is completed at the second intermediate condition cond. 12.

Performing a single one type of adjustment (for example, a first coordinate plane position adjustment, a second coordinate plane position adjustment, a rotation, a collision resolution, or a gap resolution) on a tooth may be defined as executing a one type of operation, and sequentially performing single two types of adjustments on a tooth may be defined as sequentially executing two types of operations. In an example, whether to update a first feature curve crv1 may be determined based on a number and a type of an operation.

In another example, whether to update a first feature curve crv1 may also be determined based on an adaptation between a dental characteristic parameter and the first feature curve crv1, for example, a mean value, a weighted mean value, or a variance of a first distance of a tooth.

In an example provided by the present application, a way of adjusting a dental characteristic parameter includes: adjusting a position of a tooth in a second coordinate plane.

In a specific example, a second coordinate plane includes an xy plane constructed based on a second axis y and a third axis x, as shown in FIG. 4.

For example, a position of at least one of tooth #11 to tooth #18, tooth #21 to tooth #28, tooth #31 to tooth #38, and tooth #41 to tooth #48 in a second coordinate plane may be adjusted to move towards a direction adapted to a second feature curve crv2.

A direction and an amount of a position adjustment of each tooth in a second coordinate plane may be the same or different.

In an example provided by the present application, a way of adjusting a dental characteristic parameter includes: translating a position of a tooth in a second coordinate plane towards a direction closer to a second feature curve.

In a specific example, a direction closer to a second feature curve may refer to a direction in which a feature point of a tooth approaches the second feature curve.

In an example, a second feature curve crv2 is always fixed and unchanged.

In an example, after a position adjustment of a tooth in a second coordinate plane is completed, a second feature curve crv2 is redetermined based on an adjusted dental characteristic parameter.

In an example, after one position adjustment of a tooth in a second coordinate plane and another operation are completed, a second feature curve crv2 is then redetermined based on an adjusted dental characteristic parameter.

The other operation may be rotating a tooth, adjusting a position of a tooth in a first coordinate plane, resolving a collision, or resolving a gap.

In an example, after a position adjustment of a tooth in a second coordinate plane is completed, whether to update a second feature curve crv2 and/or whether to continue to adjust a tooth position in the second coordinate plane is determined based on an adaptation between a dental characteristic parameter and the second feature curve crv2.

In another example provided by the present application, a way of adjusting a dental characteristic parameter may also include adjusting a position of a tooth in another coordinate plane such as a third coordinate plane.

In a specific example, a third coordinate plane includes an xz plane constructed based on a first axis z and a third axis x.

In an example provided by the present application, a way of adjusting a dental characteristic parameter includes: rotating a tooth.

In a specific example, rotating a tooth includes determining a rotation axis extension direction, determining a rotation axis position, and rotating a set angle around the determined rotation axis.

In a specific example, as shown in FIG. 11, a rotation axis extension direction may be a long axis ax1 direction of a tooth (a growth direction or a direction from a root to a crown), or a short axis ax2 direction of a tooth (a mesiodistal direction).

In a specific example, as shown in FIG. 11, a rotation axis extension direction may be a first axis z direction, a second axis y direction, or a third axis x direction of a dental arch coordinate system.

In a specific example, as shown in FIG. 11 to FIG. 13, a rotation axis position may pass through a geometric center of a crown of a tooth, a geometric center of an occlusal surface, a midpoint of an incisal ridge, a center of resistance, or a feature point of a tooth, a corresponding reference point refl on a first feature curve, or a corresponding base point bm1 on a second feature curve.

In an example provided by the present application, a way of adjusting a dental characteristic parameter includes: with a first normal of a first coordinate plane as a rotation axis, rotating a tooth towards a first rotation direction.

As shown in FIG. 11, in a specific example, a first axis z, a second axis y, and a third axis x are perpendicular to each other, a first coordinate plane includes a yz plane constructed based on the first axis z and the second axis y, and then the third axis x is one of normals of the first coordinate plane.

During an adjustment process from a first intermediate condition cond. 11 to a second intermediate condition cond. 12, when tooth #24 has a torsion amount, tooth #24 may be rotated towards a first rotation direction rot1 with a first normal x24 parallel to a third axis x as a rotation axis.

In a specific example, a first rotation direction is configured to make a long axis of a tooth in a first coordinate plane perpendicular to a first feature curve.

Continuing with FIG. 11, under a first intermediate condition cond. 11, an angle between a long axis ax1 of tooth #24 and a first feature curve crv1 is an acute angle; based on this, a first rotation direction rot1 may be determined as a direction for increasing the angle between the long axis ax1 of tooth #24 and the first feature curve crv1. After being rotated, tooth #24 is in a second intermediate condition cond. 12, and at this time, the long axis ax1 of tooth #24 is perpendicular to the first feature curve crv1.

Although in a technical solution shown in FIG. 11, a long axis of a tooth is made perpendicular to a first feature curve through one rotation operation, in another example, executing one rotation operation on the tooth according to a first rotation direction may only increase an acute angle between the long axis of the tooth and the first feature curve, or reduce an obtuse angle between the long axis of the tooth and the first feature curve, and is not required to be directly adjusted to be mutually perpendicular.

In an example, a determination process of a long axis of a tooth may be that a shape characteristic of the tooth is input into a pre-constructed deep learning model to predict and obtain a characteristic information of a corresponding long axis of the tooth, or may be obtained through a manual annotation.

In an example provided by the present application, a way of adjusting a dental characteristic parameter includes: rotating a tooth towards a second rotation direction.

In a specific example, a second rotation direction is configured to make a first short axis parallel to a tangent of a second feature curve.

A first short axis is a short axis of a tooth extending along a mesiodistal direction in a second coordinate plane.

As shown in FIG. 11, under a first intermediate condition cond. 11, an angle between a short axis ax2 of tooth #25 and a first feature curve crv1 is an acute angle; based on this, a second rotation direction rot2 may be determined as a direction for reducing the angle between the short axis ax2 of tooth #25 and the first feature curve crv1. After being rotated, tooth #25 is in a second intermediate condition cond. 12, and at this time, the short axis ax2 of tooth #25 is parallel to the first feature curve crv1.

In FIG. 11, for a convenience of display, a first feature curve crv1 is deformed into a straight line. It can be understood that the deformed first feature curve crv1 actually indicates a tangent at a corresponding position on a curve. Therefore, that a short axis ax2 of tooth #25 shown in FIG. 11 is parallel to the first feature curve crv1 means that: the short axis ax2 of tooth #25 is parallel to a tangent direction at a position on an actual first feature curve corresponding to tooth #25. In an example, a tangent point on the first feature curve corresponding to tooth #25 may be a reference point corresponding to tooth #25.

Although in a technical solution shown in FIG. 11, a short axis of a tooth is made parallel to a first feature curve through one rotation operation, in another example, executing one rotation operation on the tooth according to a second rotation direction may only reduce an acute angle between the short axis of the tooth and the first feature curve, or increase an obtuse angle between the short axis of the tooth and the first feature curve, and is not required to be directly adjusted to be mutually parallel.

In an example, a determination process of a short axis of a tooth may be that a shape characteristic of the tooth is input into a pre-constructed deep learning model to predict and obtain a characteristic information of a corresponding short axis of the tooth, or a position and a distribution of the short axis of the tooth are obtained through a manual annotation.

In an example provided by the present application, a way of adjusting a dental characteristic parameter includes: rotating a tooth towards a first inclination angle direction.

In a specific example, a first inclination angle direction is a direction for reducing a difference between a long axis inclination angle of a tooth and a standard long axis inclination angle of the tooth.

A long axis inclination angle is an angle between a long axis of a tooth and an extension direction of a first axis of a dental arch coordinate system.

As shown in FIG. 12, in a specific example, for tooth #26, a dental arch coordinate system includes a third coordinate plane, and the third coordinate plane includes an xz plane constructed based on a first axis z and a third axis x. A first inclination angle direction may be a direction for adjusting a tooth torque in the third coordinate plane.

In another specific example, or for another tooth, in combination with FIG. 4, a vertical axis of a third coordinate plane may be a third axis z, and a way of determining a horizontal axis may include: determining a tangent and a tangent point on a second feature curve crv2 corresponding to a tooth, taking the tangent as a normal to form a plane passing through the tangent point, and an intersection line formed by an intersection of the plane and a second coordinate plane (for example, an xy plane) forms the horizontal axis of the third coordinate plane of the tooth. In an example, a tangent point on the second feature curve corresponding to a tooth may be a base point corresponding to the tooth.

For example, for tooth #34, a corresponding second feature curve crv2 has a tangent (a bold dashed line in FIG. 4), and a horizontal axis x34 of a third coordinate plane of tooth #34 may be determined based on a perpendicular line of the tangent in a second coordinate plane, or based on an intersection line formed by the second coordinate plane and a plane formed by taking the tangent as a normal.

(a) in FIG. 12 shows a long axis inclination angle formed by tooth #26 before and after a rotation; (b) in FIG. 12 shows a process in which tooth #26 rotates towards a first inclination angle direction rot3, wherein a dashed line shows a posture before the rotation, and a solid line shows a posture after the rotation.

For tooth #26, tooth #26 has a long axis ax1 in a state, and a long axis inclination angle formed between the long axis ax1 of the tooth and a first axis z is *α*1. Tooth #26 is rotated towards a first inclination angle direction rot3, so that tooth #26 has a standard long axis ax1s, and a standard long axis inclination angle formed between the standard long axis ax1s and the first axis z is *α*2.

Before rotating towards a first inclination angle direction rot3, a difference between a long axis inclination angle of tooth #26 and a standard long axis inclination angle is (*α*1 - *α*2) *.* After a rotation adjustment, the difference between the long axis inclination angle of tooth #26 and the standard long axis inclination angle is 0.

In an example, continuing with FIG. 12, a tangent of a first feature curve crv1 at a reference point refl may be used as a rotation axis, and a first reference line z1 parallel to a first axis z is passed through the reference point refl. Based on this, an angle between a long axis ax1 of a tooth and the first reference line z1 is a long axis inclination angle *α*1, and an angle between a standard long axis ax1s and the first reference line z1 is a standard long axis inclination angle *α*2.

In some examples, a rotation axis corresponding to rotating a tooth towards a first inclination angle direction may not be along a tangent direction of a first feature curve crv1 at a reference point refl, but may be any axis passing through the reference point refl and intersecting with a third coordinate plane of the tooth.

Although in a technical solution shown in FIG. 12, a long axis inclination angle of a tooth is made equal to a standard long axis inclination angle through one rotation operation, in another example, it is not required to be directly adjusted to be equal.

In an example provided by the present application, a way of adjusting a dental characteristic parameter includes: based on a second rotation axis, rotating a tooth towards a second inclination angle direction.

A second inclination angle direction is a direction for reducing a difference between a long axis inclination angle and a standard long axis inclination angle.

A second rotation axis passes through an intersection point of a second reference line and a long axis of a tooth.

A second reference line is a reference line passing through a base point coordinate and parallel to a first axis.

A base point coordinate is a base point coordinate on a second feature curve corresponding to a feature point coordinate of a tooth.

As shown in FIG. 13, (a) in FIG. 13 shows a long axis inclination angle formed by tooth #26 before and after a rotation; (b) in FIG. 13 shows a process in which tooth #26 rotates towards a second inclination angle direction rot4, wherein a dashed line shows a posture before the rotation, and a solid line shows a posture after the rotation.

For tooth #26, tooth #26 has a long axis ax1 in a state, and a long axis inclination angle formed between the long axis ax1 of the tooth and a first axis z is *α*1 . Tooth #26 is rotated towards a second inclination angle direction rot4, so that tooth #26 has a standard long axis ax1s, and a standard long axis inclination angle formed between the standard long axis ax1s and the first axis z is α2 .

Before rotating towards a second inclination angle direction rot4, a difference between a long axis inclination angle of tooth #26 and a standard long axis inclination angle is (*α*1 - *α*2). After a rotation adjustment, the difference between the long axis inclination angle of tooth #26 and the standard long axis inclination angle is 0.

Continuing with FIG. 13, there exists a base point bm1 corresponding to tooth #26 on a second feature curve crv2, and a second reference line z2 parallel to a first axis z is passed through the base point bm1. Based on this, an angle between a long axis ax1 of a tooth and the second reference line z2 is a long axis inclination angle *α*1, and an angle between a standard long axis ax1s and the second reference line z2 is a standard long axis inclination angle *α*2.

A long axis ax1 of a toothintersects with a second reference line z2 at a second rotation point r2, and a second rotation axis corresponding to rotating a tooth towards a second inclination angle direction rot4 may be any axis passing through the second rotation point r2.

In a specific example, an extension direction of a second rotation axis intersects with a third coordinate plane corresponding to tooth #26.

In a specific example, an extension direction of a second rotation axis is parallel to a tangent of a second feature curve crv2 at a base point bm1.

Although in a technical solution shown in FIG. 13, a long axis inclination angle of a tooth is made equal to a standard long axis inclination angle through one rotation operation, in another example, it is not required to be directly adjusted to be equal.

FIG. 12 and FIG. 13 provide two technical solutions for adjusting a long axis inclination angle of a tooth and improving a tooth torque. The two solutions both eventually adjust the long axis inclination angle of the tooth to a standard long axis inclination angle, but are different in a rotation axis. A rotation axis in a technical solution provided in FIG. 13 is farther from a tooth than a rotation axis in a technical solution provided in FIG. 12, and therefore when a rotation adjustment is performed to a same standard long axis inclination angle, the technical solution corresponding to FIG. 13 causes the tooth to generate more movement amount in a third coordinate plane.

Although in an example disclosed in FIG. 12 and FIG. 13, a first feature curve crv1 and a second feature curve crv2 are at different positions of tooth #26 in a third coordinate plane, and a reference point refl and a base point bm1 are at different positions of tooth #26. However, in another example, in the third coordinate plane, the first feature curve crv1 and the second feature curve crv2 may overlap, the reference point refl and the base point bm1 may overlap, and at this time, a first inclination angle direction rot3 is the same as a second inclination angle direction rot4, and a corresponding rotation axis is also the same.

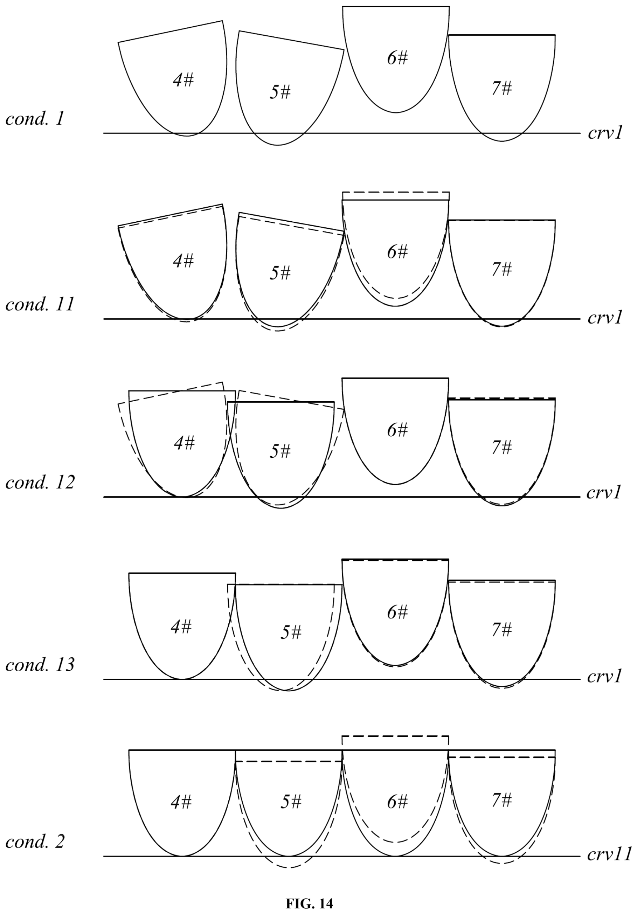

FIG. 14 shows an adjustment process of tooth #4 to tooth #7 from a first condition cond. 1 to a second condition cond. 2, wherein a dashed line represents a position and a posture of a tooth in a previous state, and a solid line represents a position and a posture of a tooth in a current state.

In an example provided by the present application, a way of adjusting a dental characteristic parameter includes: resolving a collision between adjacent teeth.

Under a second intermediate condition cond. 12 in FIG. 14, a rotated tooth #4 collides with tooth #5.

In an example, a collision is expressed as that an overlapping area size of a point cloud data distribution of tooth #4 and a point cloud data distribution of tooth #5 exceeds an allowable error range.

Based on this, under a third intermediate condition cond. 13 in FIG. 14, tooth #5 may be adjusted to move in a direction away from tooth #4, so as to resolve a collision between the two teeth. A movement includes a translation, a rotation, or a deformation.

In an example provided by the present application, a way of adjusting a dental characteristic parameter includes: resolving a gap between adjacent teeth.

Under a second intermediate condition cond. 12 in FIG. 14, a gap exists between a rotated tooth #5 and tooth #6.

In an example, an existence of a gap is expressed as that an average distance, a weighted average distance, a maximum distance, or a minimum distance of a point cloud data distribution of tooth #5 and a point cloud data distribution of tooth #6 at an adjacent position exceeds an allowable error range.

Based on this, at a third intermediate condition cond. 13 in FIG. 14, tooth #5 may be adjusted to move in a direction close to tooth #6, so as to resolve a gap between the two teeth. A movement includes a translation, a rotation, or a deformation.

In an example provided by the present application, a method for determining an orthodontic reference curve includes: successively adjusting a dental characteristic parameter of a tooth to be adjusted with an adjustment step length, and updating a first feature curve accordingly.

In an example where a tooth adjustment is implemented through a rotation, an angle step length for rotating a tooth towards a corresponding direction may be 0.1°, 0.5°, 1°, or set according to a need.

In an example where a tooth adjustment is implemented through a translation, a translation step length for moving a tooth towards a corresponding direction may be determined based on a total distance and a preset proportional coefficient.

In a specific example, a preset proportional coefficient is a real number greater than or equal to 0 and less than or equal to 1, and a translation step length is a product of the proportional coefficient and a total distance.

For example, in combination with FIG. 8, a first distance d1 between tooth #26 and a first feature curve crv1 is a total distance, and therefore a translation step length of tooth #26 may be set as a product of the first distance d1 and a preset proportional coefficient, thereby successively adjusting tooth #26 to tend to adapt to the first feature curve crv1.

In an example, a tooth is adjusted once according to an adjustment step length, that is, a first feature curve is updated based on an adjusted dental characteristic parameter. At this time, adjusting the tooth once according to the adjustment step length may be regarded as performing one operation on the tooth as described above.

In another example, a first feature curve may also be updated based on a dental characteristic parameter after one operation of a plurality of combinations, a plurality of operations of a single combination, or a plurality of operations of a plurality of combinations is performed on a tooth.

In combination with FIG. 14, during an adjustment process from a first condition cond. 1 to a first intermediate condition cond. 11, tooth #4, tooth #5, and tooth #6 move in a direction close to a first feature curve crv1.

During an adjustment process from a first intermediate condition cond. 11 to a second intermediate condition cond. 12, tooth #4 and tooth #5 are rotated to adapt to a first feature curve crv1 in terms of an inclination degree.

During an adjustment process from a second intermediate condition cond. 12 to a third intermediate condition cond. 13, tooth #5 resolves a collision and a gap with an adjacent tooth through a movement.

During an adjustment process from a third intermediate condition cond. 13 to a second condition cond. 2, tooth #5, tooth #6, and tooth #7 are moved and aligned under a constraint such as avoiding a collision and a gap, and at this time, a first feature curve is updated based on a redetermined dental characteristic parameter. Since at this time, a deviation amount of tooth #4, tooth #5, tooth #6, and tooth #7 relative to the updated first feature curve satisfies an allowable error range, the updated first feature curve at this time is determined as a second orthodontic reference curve crv11.

In an example provided by the present application, a method for determining an orthodontic reference curve includes: successively adjusting a dental characteristic parameter of a tooth to be adjusted with an adjustment step length, and updating a second feature curve accordingly.

In an example where a tooth adjustment is implemented through a rotation, an angle step length for rotating a tooth towards a corresponding direction may be 0.1°, 0.5°, 1°, or set according to a need.

In an example where a tooth adjustment is implemented through a translation, a translation step length for moving a tooth towards a corresponding direction may be determined based on a total distance and a preset proportional coefficient.

In an example, a tooth is adjusted once according to an adjustment step length, that is, a second feature curve is updated based on an adjusted dental characteristic parameter. At this time, adjusting the tooth once according to the adjustment step length may be regarded as performing one operation on the tooth as described above.

In another example, a second feature curve may also be updated based on a dental characteristic parameter after one operation of a plurality of combinations, a plurality of operations of a single combination, or a plurality of operations of a plurality of combinations is performed on a tooth.

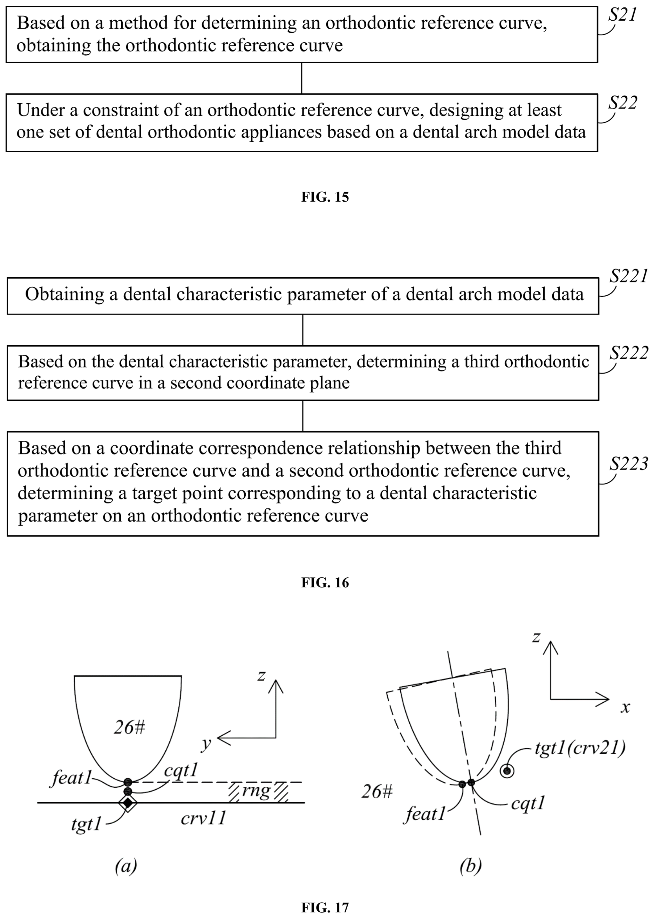

As shown in FIG. 15, an embodiment of the present application provides a method for designing a dental orthodontic appliance.

An application or an instruction corresponding to the method may be loaded on an electronic device, an apparatus for designing a dental orthodontic appliance, and/or a computer storage medium, may be loaded on a carrier of a display method provided below, and may be loaded on a carrier for implementing a dental orthodontic appliance preparation, to achieve a corresponding technical effect.

A method for designing a dental orthodontic appliance may specifically include the following steps.

Step S21, based on a method for determining an orthodontic reference curve, obtaining the orthodontic reference curve.

In an example, a method for determining an orthodontic reference curve may include at least one of the following steps:
obtaining a dental characteristic parameter of a dental arch model data;
based on the dental characteristic parameter, determining a first feature curve in a first coordinate plane and a second feature curve in a second coordinate plane, the first feature curve being a first orthodontic reference curve in a dental arch coordinate system; and
adjusting the dental characteristic parameter, an adjusted dental characteristic parameter being configured to determine a second orthodontic reference curve, and the adjusted dental characteristic parameter is adapting to a corresponding first feature curve and a corresponding second feature curve.

The method for determining an orthodontic reference curve may be any method provided in a preceding embodiment.

In combination with FIG. 1, when designing a dental orthodontic appliance 100, a second orthodontic reference curve crv11 may be first determined based on a dental arch model data, and then the dental orthodontic appliance 100 is designed accordingly.

In combination with FIG. 4, in an example, a second feature curve crv2 may also be used as an orthodontic reference curve to provide a reference for a design of a dental orthodontic appliance. In a specific example, the second feature curve crv2 is redetermined as a dental characteristic parameter is adjusted, and an updated second feature curve crv2 may be used for designing a dental orthodontic appliance. In a specific example, the second feature curve crv2 is a feature curve corresponding to an original dental arch model and does not change as the dental characteristic parameter is adjusted, and this type of second feature curve crv2 may be used for designing a dental orthodontic appliance.

A second feature curve crv2 used for providing an orthodontic reference is defined as a third orthodontic reference curve crv21 below.

In a specific example, an orthodontic reference curve in step S21 simultaneously includes a second orthodontic reference curve crv11 and a third orthodontic reference curve crv21.

Step S22, under a constraint of an orthodontic reference curve, designing at least one set of dental orthodontic appliances based on a dental arch model data.

In an example, a constraint specifically includes: with an orthodontic reference curve as an orthodontic target, designing a dental orthodontic appliance, so that an orthodontically treated tooth is adapted to the orthodontic reference curve.

In an example, a constraint specifically includes: setting a dental orthodontic space according to an orthodontic reference curve, and designing a dental orthodontic appliance, so that a tooth in an orthodontic process does not leave the orthodontic space (for example, the tooth at any position in the orthodontic process is located in the orthodontic space).

In an example, a constraint specifically includes: based on a difference between an orthodontic reference curve and a current tooth arrangement, determining an orthodontic effect of a current dental orthodontic appliance.

In an example, a constraint specifically includes: designing a dental orthodontic appliance based on a second orthodontic reference curve, and maintaining a tooth to be orthodontically treated towards a direction adapted to a third orthodontic reference curve, or maintaining the tooth not to leave an orthodontic space set by the third orthodontic reference curve (for example, the tooth at any position in an orthodontic process is located in the orthodontic space).

In an example, a designed dental orthodontic appliance satisfies a condition: causing a tooth to be orthodontically treated towards a direction adapted to an orthodontic reference curve.

In an example, a dental orthodontic appliance sets an orthodontic point of a tooth, and in a same dental arch coordinate system, the orthodontic point is located on a side of a feature point of a corresponding tooth in a dental arch model closer to an orthodontic reference curve.

(a) in FIG. 17 shows a solution for setting an orthodontic point based on a second orthodontic reference curve crv11 when a tooth position orthodontic treatment is performed.

Tooth #26 has a feature point feat1, and a second orthodontic reference curve crv11 is located on one side of the feature point feat1. Based on this, tooth #26 may be orthodontically treated through a design of a dental orthodontic appliance, so that the feature point feat1 of tooth #26 is adapted to the second orthodontic reference curve crv11, or a difference between the feature point feat1 of tooth #26 and the second orthodontic reference curve crv11 is within an allowable error range.

A dental orthodontic appliance used for achieving an orthodontic treatment of tooth #26 may be configured as a single one, and at this time, the dental orthodontic appliance directly sets a target point tgtl on a second orthodontic reference curve crv11 corresponding to a feature point feat1 as an orthodontic point.

A dental orthodontic appliance used for achieving an orthodontic treatment of tooth #26 may be configured with a plurality of ones, and at this time, the dental orthodontic appliance sets a point between a second orthodontic reference curve crv11 and a feature point feat1 as an orthodontic point cqtl. In this way, the dental orthodontic appliance first moves tooth #26 from the feature point feat1 thereof to the orthodontic point cqtl, and then moves tooth #26 from the orthodontic point cqt1 thereof to be adapted to the second orthodontic reference curve crv11, so as to gradually complete the orthodontic treatment.

As shown in (a) in FIG. 17, after a target point tgt1 corresponding to a feature point feat1 is determined, an orthodontic point cqtl may be limited to be distributed between the feature point feat1 and the target point tgt1 based on a position of the feature point feat1 and a position of the target point tgtl, so as to ensure that tooth #26 at least does not have a stage of moving in a direction away from a second orthodontic reference curve crv11 relative to an initial position of tooth #26 during an orthodontic process.

In an example, a method for designing a dental orthodontic appliance includes steps: obtaining a target point corresponding to a dental characteristic parameter of a tooth in a dental arch model on an orthodontic reference curve, and obtaining and, with a first axis coordinate of the target point, constraining a setting range of an orthodontic point of a corresponding tooth in a first axis direction.

In combination with (a) in FIG. 17, when a positive direction of a first axis z is from a second orthodontic reference curve crv11 towards tooth #26, a coordinate value of an orthodontic point cqt1 on the first axis z may be set to be less than a coordinate value of a feature point feat1 on the first axis z and greater than a coordinate value of a target point tgtl on the first axis z. Thus, a setting range rng of the orthodontic point in a first axis direction is formed.

In some examples, a similar setting range constraint may also be made on a second axis y and a third axis x based on a third orthodontic reference curve.

In an example, a reference line may be formed along an axis passing through a feature point of a tooth, and an intersection point of the reference line and an orthodontic reference curve is used as a target point of the tooth.

As shown in (a) in FIG. 17, a reference line may be formed along a first axis z passing through a feature point feat1 of tooth #26, and intersects with a second orthodontic reference curve crv11 at a target point tgt1.

(b) in FIG. 17 shows a solution for setting an orthodontic point cqtl based on a third orthodontic reference curve crv21 when a tooth torque orthodontic treatment is performed.

An orthodontic point cqtl is located on a side of a feature point feat1 closer to a third orthodontic reference curve crv21; the orthodontic point cqtl is located on a side of the feature point feat1 closer to a target point tgtl.

As shown in (b) in FIG. 17, a reference line may be formed along a third axis x (or another horizontal axis of a third coordinate plane of a tooth) passing through tooth #26, and intersects with a third orthodontic reference curve crv21 at a target point tgtl.

In another specific example, a reference line may also be formed along a second axis y to determine a target point.

In an example, based on a position of a tooth in one coordinate plane, a point on an orthodontic reference curve in another coordinate plane adapted to the position is determined as a target point of the tooth.

As shown in FIG. 17, for example, in a third coordinate plane, tooth #26 has a first axis z coordinate value and a third axis x coordinate value, and tooth #26 has been adjusted to a target position in the third coordinate plane, and then a point with a same first axis z coordinate value may be determined on a second orthodontic reference curve crv11 in a first coordinate plane, and is used as a target point tgt1 on the second orthodontic reference curve crv11 corresponding to tooth #26.

In another example, in combination with (a) in FIG. 17 and FIG. 4, in a second coordinate plane, tooth #26 has a second axis y coordinate value and a third axis x coordinate value, and tooth #26 has been adjusted to a target position in the second coordinate plane, and then a point with a same second axis y coordinate value may be determined on a second orthodontic reference curve crv11 in a first coordinate plane, and is used as a target point tgtl on the second orthodontic reference curve crv11 corresponding to tooth #26.

In some other examples, a target point on a third orthodontic reference curve crv21 in a second coordinate plane corresponding to a tooth may also be determined based on a coordinate value of the tooth in a first coordinate plane.

In an example, based on a position relationship between a tooth and one orthodontic reference curve, a point on another orthodontic reference curve adapted to the position relationship is determined as a target point of the tooth.

A position relationship may be a Euclidean distance from a feature point of a tooth to an orthodontic reference curve, or may be a difference between a coordinate value of the feature point of the tooth and a coordinate value of a foot of a perpendicular from the feature point of the tooth to the orthodontic reference curve.

For example, a position relationship between a feature point of a tooth and a third orthodontic reference curve crv21 may be determined. Since a plane where the third orthodontic reference curve crv21 is located and a plane where a second orthodontic reference curve crv11 is located have a common second axis y, therefore, a position relationship between the feature point of the tooth and the second orthodontic reference curve crv11 may be determined by using the second axis y as a medium based on the above position relationship, so as to determine a target point of the tooth.

In an example, as shown in FIG. 16, a method for designing a dental orthodontic appliance includes at least one of the following steps:
step S221, obtaining a dental characteristic parameter of a dental arch model data;
step S222, based on the dental characteristic parameter, determining a third orthodontic reference curve in a second coordinate plane; and
step S223, based on a coordinate correspondence relationship between the third orthodontic reference curve and a second orthodontic reference curve, determining a target point corresponding to a dental characteristic parameter on an orthodontic reference curve.

In combination with FIG. 4 and (a) in FIG. 17, if a third orthodontic reference curve is a second feature curve crv2 corresponding to an original dental arch model and does not change as a tooth is adjusted, then in a second coordinate plane, a feature point of the tooth is on the third orthodontic reference curve, and a second axis y coordinate value and a third axis x coordinate value when the feature point of the tooth is adapted to the third orthodontic reference curve may be determined.

Based on a second axis y coordinate value, a corresponding point on a second orthodontic reference curve crv11 in a first coordinate plane is determined, and the corresponding point is a target point of a feature point of a tooth in the first coordinate plane.

Conversely, a target point on a third orthodontic reference curve may also be determined based on a second orthodontic reference curve crv11.

In combination with FIG. 17, if a third orthodontic reference curve is different from a second feature curve of an original dental arch model and changes as a tooth is adjusted, then in a second coordinate plane or a third coordinate plane, a relative position relationship exists between a feature point of the tooth and a corresponding point on the third orthodontic reference curve, and the relative position relationship may be expressed as a difference of second axis y coordinate values of the feature point and the corresponding point or a difference of first axis z coordinate values of the feature point and the corresponding point.

A target point on a second orthodontic reference curve crv11 in a first coordinate plane may also be determined based on a difference of coordinate values.

Conversely, a target point on a third orthodontic reference curve may also be determined based on a second orthodontic reference curve crv11.

In an example, steps S221 to S223 are executed in sequence.

In an example, steps S221 to S223 are included in step S22.

In an example provided by the present application, a method for designing a dental orthodontic appliance includes: obtaining a target point corresponding to a dental characteristic parameter of a tooth in a dental arch model on an orthodontic reference curve, obtaining a normal vector of a tangent at the target point on an orthodontic reference curve, determining an axial inclination amount based on the normal vector and a long axis of a corresponding tooth, and setting at least one orthodontic point corresponding to the tooth based on the axial inclination amount.

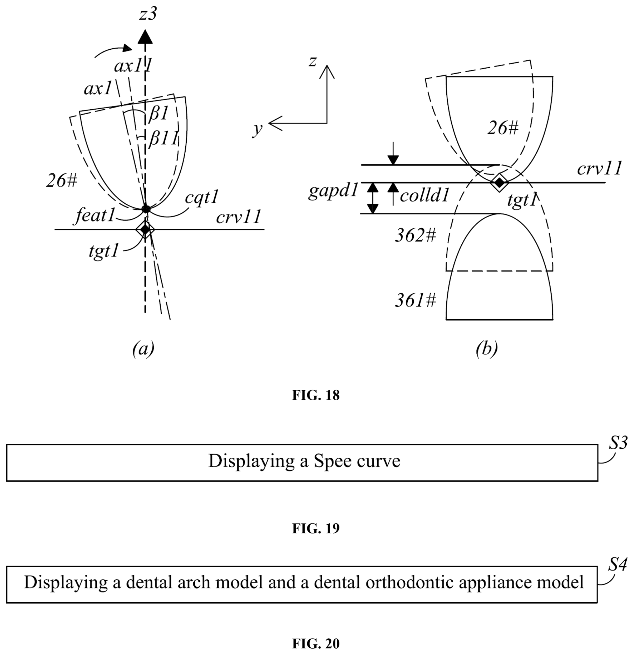

As shown in (a) in FIG. 18, in an example, an "orthodontic point" set by a dental orthodontic appliance simultaneously includes an orthodontic point cqtl corresponding to a feature point feat1, and a post-orthodontic long axis ax11 or a torsion inclination angle *β*11.

Due to a deformation, a second orthodontic reference curve crv11 shown in (a) in FIG. 18 is in a shape of a tangent of the second orthodontic reference curve at a target point tgtl. A normal vector of the tangent may be formed in a first coordinate plane. Assuming that the normal vector is parallel to a first axis z, a third normal vector z3 may be obtained.

A dashed line part in (a) in FIG. 18 shows tooth #26 before an orthodontic treatment, and tooth #26 has a long axis axl. An angle between the long axis ax1 and a third normal vector z3 is a torsion inclination angle *β*1, and is used to describe an axial inclination amount of a tooth.

With a target of making a long axis of a tooth perpendicular to a tangent of a second orthodontic reference curve crv11, or making a short axis of a tooth parallel to a tangent of a second orthodontic reference curve crv11, tooth #26 may be rotated. In this process, an orthodontic point cqtl corresponding to a feature point feat1, and a post-orthodontic long axis ax11 or a post-orthodontic torsion inclination angle *β*11 may be determined based on a factor such as a preset step length, a collision, a gap, a root resorption, and a resistance, and then a dental orthodontic appliance is designed accordingly by using the orthodontic point cqtl, the post-orthodontic long axis ax11, and/or the post-orthodontic torsion inclination angle *β*11 as one state of an orthodontic process.

In an example provided by the present application, a method for designing a dental orthodontic appliance includes: obtaining a target point corresponding to a dental characteristic parameter of a tooth in a dental arch model on an orthodontic reference curve, obtaining a normal vector of a tangent at the target point on an orthodontic reference curve, and determining an occlusal depth parameter between the tooth and an opposing tooth along the normal vector to constrain a setting range of an orthodontic point of a corresponding tooth in a direction of the normal vector.

(b) in FIG. 18 shows an occlusal depth parameter determined in two different situations. For tooth #26, a dashed line part is an initial posture of tooth #26 without an orthodontic treatment, and a solid line part is a target posture after an orthodontic treatment corresponding to a target point tgtl of tooth #26. In combination with FIG. 4, tooth #361 shows a posture of tooth #36 in a first situation, and tooth #362 shows a posture of tooth #36 in a second situation. Tooth #36 and tooth #26 are opposing teeth.

Regarding opposing teeth, in combination with FIG. 4, a pair of teeth with a same number (for example, both being tooth #4) located in a quadrant I (pointing to an upper jaw) and a quadrant IV (pointing to a lower jaw) may be defined as opposing teeth, for example, tooth #14 and tooth #44. A pair of teeth with a same number (for example, both being tooth #4) located in a quadrant II (pointing to an upper jaw) and a quadrant III (pointing to a lower jaw) may be defined as opposing teeth, for example, tooth #24 and tooth #34.

When an occlusion is normal, an occlusal relationship exists between opposing teeth.

Continuing with (b) in FIG. 18, if a normal vector of a tangent at a target point tgt1 on a second orthodontic reference curve crv11 is a unit vector in a first axis z direction, an occlusal depth parameter may be determined along a positive direction or a negative direction of the first axis z.

In a first situation, tooth #26 has an opposing tooth #361. Since tooth #26 points to an upper jaw, a first relative point located on tooth #361 is determined from tooth #26 towards a negative direction of a first axis z, and a distance between the first relative point and a target point tgtl is determined as a first gap value gapdl. In this way, the first gap value may be defined as a positive value, and it is determined that an occlusal gap exists between tooth #26 and the opposing tooth #361 thereof, and a gap value is the first gap value gapd1.

In a second situation, tooth #26 has an opposing tooth #362. Since tooth #26 points to an upper jaw, when a relative point cannot be determined towards a negative direction of a first axis z, a second relative point located on tooth #362 is determined from tooth #26 towards a positive direction of the first axis z, and a distance between the second relative point and a target point tgtl is determined as a first collision value colldl. In this way, the first collision value may be defined as a negative value, and it is determined that an occlusal collision exists between tooth #26 and the opposing tooth #362 thereof, and a collision depth is the first collision value colld1.

As shown in FIG. 19, an embodiment of the present application provides a display method.

An application or an instruction corresponding to the method may be loaded on an electronic device, an apparatus for determining an orthodontic reference curve, and/or a computer storage medium, may be loaded on a carrier for implementing a dental orthodontic appliance preparation, and may also be loaded in an apparatus for designing a dental orthodontic appliance, to achieve a corresponding technical effect.

The display method may specifically include:
step S3, displaying a Spee curve.

In an example, a Spee curve is obtained based on a method for determining an orthodontic reference curve.

In a specific example, a Spee curve is obtained based on a method for determining an orthodontic reference curve provided in any preceding embodiment.

In a specific example, a preceding second orthodontic reference curve may be a Spee curve.

In a specific example, a preceding first feature curve may be a Spee curve.

In a specific example, a preceding first orthodontic reference curve may be a Spee curve.

In an example, a display method is used for displaying a static Spee curve.

In an example, a display method is used for displaying a dynamic Spee curve. Specifically, a process in which a Spee curve changes as a dental characteristic parameter is adjusted may be displayed.

At this time, a display method may sequentially display a first orthodontic reference curve, a different first feature curve determined according to a different dental characteristic parameter, and a second orthodontic reference curve.

In an example, a display method includes: displaying a dental arch model. In this way, a comparison between the dental arch model and a Spee curve may be provided.

In an example, a display method includes: displaying a dental orthodontic appliance model. In this way, a comparison between the dental orthodontic appliance model and a Spee curve may be provided.

In an example, a Spee curve is used for designing an orthodontic plan.

In a specific example, a display method, in response to an adjustment of a position, a posture, and a shape of a tooth, a Spee curve, a dental arch curve, a dental orthodontic appliance, etc. by a user, includes a step: outputting a corresponding display content.

In an example, a display method includes: displaying a second feature curve.

In an example, a display method includes: displaying a third orthodontic reference curve.

In an example, a display method includes: displaying a dental arch curve.

In an example, a display method includes: displaying a point, a line, a plane, a body, or a value involved in a process of redetermining a Spee curve based on a dental characteristic parameter. For example, a feature point, a reference point, a first distance, a reference line, a long axis of a tooth, a short axis of a tooth, a rotation direction, a base point, and an angle.

In an example, a display method includes: displaying a point, a line, a plane, a body, or a value involved in a process of designing a treatment plan based on a Spee curve. For example, a feature point, a target point, an orthodontic point, a setting range, a long axis, a short axis, an angle, a gap value, and a collision value.

As shown in FIG. 20, an embodiment of the present application provides a display method.

An application or an instruction corresponding to the method may be loaded on an electronic device, an apparatus for designing a dental orthodontic appliance, and/or a computer storage medium, may be loaded on a carrier for implementing a dental orthodontic appliance preparation, to achieve a corresponding technical effect.

The display method may specifically include:
step S4, displaying a dental arch model and a dental orthodontic appliance model.

In an example, a dental orthodontic appliance model is obtained based on a method for designing a dental orthodontic appliance.

In a specific example, a dental orthodontic appliance model is obtained based on a method for designing a dental orthodontic appliance provided in any preceding embodiment.

In an example, a display method is used for displaying a static dental arch model and a static dental orthodontic appliance model.

In an example, a display method is used for displaying a dynamic dental arch model and a dynamic dental orthodontic appliance model. Specifically, a process in which a dental arch model and/or a dental orthodontic appliance model changes as an orthodontic stage develops may be displayed.

In an example, a display method includes: displaying an orthodontic reference curve. In this way, a comparison among a dental arch model, a dental orthodontic appliance model, and the orthodontic reference curve may be provided.

In a specific example, an orthodontic reference curve includes: at least one of a first orthodontic reference curve, a second orthodontic reference curve, and a third orthodontic reference curve.

In an example, a display method includes: displaying a feature curve.

In a specific example, an orthodontic reference curve includes: at least one of a first feature curve at each stage and a second feature curve at each stage.

In a specific example, a display method, in response to an adjustment of a position, a posture, and a shape of a tooth, an orthodontic reference curve, a feature curve, a dental orthodontic appliance, etc. by a user, outputs a corresponding display content.

In an example, a display method includes: displaying a point, a line, a plane, a body, or a value involved in a process of designing a treatment plan based on a Spee curve.

In summary, the method and the apparatus for determining a reference curve, the dental orthodontic appliance, and the design method provided by the present application, after determining two feature curves for a dental arch model, adjust a tooth to adapt to the feature curves, and determine an orthodontic reference curve based on the adjusted tooth. This enables an automatic determination of an orthodontic reference curve based on a given dental arch model and achieves a customized configuration of the orthodontic reference curve for a different dental arch model; providing two feature curves in two coordinate planes as a constraint allows the orthodontic reference curve determined by the method to simultaneously satisfy an actual situation in at least two dimensions, improving feasibility and accuracy, and saving unnecessary time consumption.

It should be understood that although this specification is described in terms of an embodiment, not every embodiment contains only one independent technical solution. This narrative method of the specification is merely for clarity. A person of ordinary skill in the art should regard the specification as a whole, and technical solutions in various embodiments may also be appropriately combined to form another embodiment understandable to the person of ordinary skill in the art.

The series of detailed descriptions listed above are merely specific explanations of feasible embodiments of the present application, and are not intended to limit a scope of protection of the present application. Any equivalent embodiment or modification made without departing from a technical spirit of the present application should be included within the scope of protection of the present application.

## Claims

1. A method for determining an orthodontic reference curve, **characterized in that** the method comprises:
obtaining dental characteristic parameters of a dental arch model data;
determining, based on the dental characteristic parameters, a first feature curve in a first coordinate plane and a second feature curve in a second coordinate plane, the first feature curve being a first orthodontic reference curve in a dental arch coordinate system, and the second feature curve being a dental arch curve; and
adjusting the dental characteristic parameters, the adjusted dental characteristic parameters being configured to determine a second orthodontic reference curve, and the adjusted dental characteristic parameters adapting to a corresponding first feature curve and a corresponding second feature curve.

2. The method for determining an orthodontic reference curve according to claim 1, **characterized in that** the method comprises:
determining, based on the first feature curve and the dental characteristic parameters, at least one first distance of at least one tooth, the first distance being a distance of the at least one tooth relative to the first feature curve;
determining a tooth to be adjusted based on the at least one first distance; and
adjusting the dental characteristic parameters based on the first distance of the tooth to be adjusted.

3. The method for determining an orthodontic reference curve according to claim 1 or 2, **characterized in that** the method specifically comprises:
determining, based on the first feature curve and the dental characteristic parameters, a plurality of first distances of a plurality of teeth relative to the first feature curve, the first distance being a distance of a tooth relative to the first feature curve;
determining a tooth satisfying a dispersion threshold condition as a tooth to be adjusted based on a dispersion degree among the plurality of first distances; or
determining a tooth to be adjusted based on sequence information of the plurality of first distances.

4. The method for determining an orthodontic reference curve according to claim 1 or 2, **characterized in that** the method specifically comprises:
determining at least one first distance of at least one tooth based on feature point coordinate information and reference point coordinate information, the feature point coordinate information being configured to indicate a feature point coordinate of the at least one tooth, and the reference point coordinate information being configured to indicate a reference point coordinate on the first feature curve corresponding to the feature point coordinate of the at least one tooth.

5. The method for determining an orthodontic reference curve according to claim 1 or 2, **characterized in that** the method comprises:
determining a reference point coordinate based on a Euclidean distance between the first feature curve and a feature point coordinate of a tooth, the reference point coordinate being configured to adjust the dental characteristic parameters.

6. The method for determining an orthodontic reference curve according to claim 1 or 2, **characterized in that** the method comprises:
determining, based on the first feature curve, a reference point coordinate in a vertical direction of a dental arch coordinate system of a tooth, the reference point coordinate being configured to adjust the dental characteristic parameters.

7. The method for determining an orthodontic reference curve according to claim 6, **characterized in that** the method specifically comprises:
determining the reference point coordinate based on the first feature curve and a first reference line, the first reference line being a reference line passing through a feature point of a tooth and extending along a direction of a first axis, the first axis being a coordinate axis of the dental arch coordinate system, and the first axis not being located in the second coordinate plane.

8. The method for determining an orthodontic reference curve according to claim 1 or 2, **characterized in that** a way of adjusting the dental characteristic parameters comprises at least one of:
adjusting a position of a tooth in the first coordinate plane;
adjusting a position of a tooth in the second coordinate plane;
rotating a tooth;
resolving a collision between adjacent teeth; and
resolving a gap between adjacent teeth.

9. The method for determining an orthodontic reference curve according to claim 1 or 2, **characterized in that** a way of adjusting the dental characteristic parameters comprises at least one of:
translating a position of a tooth in the first coordinate plane towards a direction closer to the first feature curve;
translating a position of a tooth in the second coordinate plane towards a direction closer to the second feature curve;
rotating a tooth towards a first rotation direction with a first normal of the first coordinate plane as a rotation axis, the first rotation direction being configured to make a long axis of the tooth in the first coordinate plane perpendicular to the first feature curve;
rotating a tooth towards a second rotation direction, the second rotation direction being configured to make a first short axis parallel to a tangent of the second feature curve, the first short axis being a short axis of the tooth extending along a mesiodistal direction in the second coordinate plane;
rotating a tooth towards a first inclination angle direction, the first inclination angle direction being a direction for reducing a difference between a long axis inclination angle of the tooth and a standard long axis inclination angle of the tooth, the long axis inclination angle being an angle between a long axis of the tooth and an extension direction of a first axis of the dental arch coordinate system; and
rotating a tooth towards a second inclination angle direction based on a second rotation axis, the second inclination angle direction being a direction for reducing a difference between a long axis inclination angle and a standard long axis inclination angle, the second rotation axis passing through an intersection point of a second reference line and a long axis of the tooth, the second reference line being a reference line passing through a base point coordinate and parallel to a first axis, the base point coordinate being a base point coordinate on the second feature curve corresponding to a feature point coordinate of the tooth.

10. The method for determining an orthodontic reference curve according to claim 1 or 2, **characterized in that** the dental characteristic parameters comprise at least one of:
an incisal ridge midpoint coordinate, a crown center coordinate, and a center of resistance coordinate of a tooth.

11. The method for determining an orthodontic reference curve according to claim 1 or 2, **characterized in that** the method comprises:
successively adjusting a dental characteristic parameter of a tooth to be adjusted with an adjustment step length, and updating the first feature curve accordingly.

12. The method for determining an orthodontic reference curve according to claim 1 or 2, **characterized in that** the method comprises:
successively adjusting a dental characteristic parameter of a tooth to be adjusted with an adjustment step length, and updating the second feature curve accordingly.

13. A method for designing a dental orthodontic appliance, **characterized in that** the method comprises:
obtaining an orthodontic reference curve based on a method for determining an orthodontic reference curve according to any one of claims 1-12; and
designing, under a constraint of the orthodontic reference curve, at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in a dental arch model closer to the orthodontic reference curve.

14. The method for designing a dental orthodontic appliance according to claim 13, **characterized in that** the method for designing a dental orthodontic appliance specifically comprises at least one of:
obtaining a target point corresponding to a dental characteristic parameter of a tooth in the dental arch model on an orthodontic curve, obtaining a normal vector of a tangent at the target point on the orthodontic reference curve, determining an axial inclination amount based on the normal vector and a long axis of a corresponding tooth, and setting at least one orthodontic point corresponding to the tooth based on the axial inclination amount; or,
obtaining a target point corresponding to a dental characteristic parameter of a tooth in the dental arch model on an orthodontic curve, and obtaining and, with a first axis coordinate of the target point, constraining a setting range of an orthodontic point of a corresponding tooth in a first axis direction; or,
obtaining a target point corresponding to a dental characteristic parameter of a tooth in the dental arch model on an orthodontic curve, obtaining a normal vector of a tangent at the target point on the orthodontic reference curve, and determining an occlusal depth parameter between the tooth and an opposing tooth along the normal vector to constrain a setting range of an orthodontic point of a corresponding tooth in a direction of the normal vector.

15. The method for designing a dental orthodontic appliance according to claim 14, **characterized in that** the method for designing a dental orthodontic appliance specifically comprises:
obtaining a dental characteristic parameter of a dental arch model data;
determining a third orthodontic reference curve in a second coordinate plane based on the dental characteristic parameter; and
determining, based on a coordinate correspondence relationship between the third orthodontic reference curve and a second orthodontic reference curve, a target point corresponding to the dental characteristic parameter on the orthodontic reference curve.

16. A display method, **characterized by** comprising:
displaying a Spee curve; the Spee curve is obtained based on a method for determining an orthodontic reference curve according to any one of claims 1-12; and
the Spee curve is used for designing an orthodontic plan.

17. A display method, **characterized by** comprising:
displaying a dental arch model and a dental orthodontic appliance model; and
the dental orthodontic appliance model is obtained based on a method for designing a dental orthodontic appliance according to any one of claims 13-15.

18. A dental orthodontic appliance, **characterized in that** the dental orthodontic appliance is prepared based on an orthodontic reference curve determined by a method for determining an orthodontic reference curve according to any one of claims 1-12, or is prepared based on a method for designing a dental orthodontic appliance according to any one of claims 13-15.

19. A computer storage medium, on which an application is stored, **characterized in that** when the application is executed, steps of a method for determining an orthodontic reference curve according to any one of claims 1-12 are implemented, or steps of a method for designing a dental orthodontic appliance according to any one of claims 13-15 are implemented.

20. An apparatus for determining an orthodontic reference curve, **characterized by** comprising:
a first module, configured to obtain a dental characteristic parameter of a dental arch model data;
a second module, configured to, based on the dental characteristic parameter, determine a first feature curve in a first coordinate plane and a second feature curve in a second coordinate plane, the first feature curve being a first orthodontic reference curve in a dental arch coordinate system, and the second feature curve being a dental arch curve; and
a third module, configured to adjust the dental characteristic parameter, an adjusted dental characteristic parameter being configured to determine a second orthodontic reference curve, and the adjusted dental characteristic parameter adapting to a corresponding first feature curve and a corresponding second feature curve.

21. An apparatus for designing a dental orthodontic appliance, **characterized by** comprising:
a fourth module, configured to obtain an orthodontic reference curve based on a method for determining an orthodontic reference curve according to any one of claims 1-12; and
a fifth module, configured to, under a constraint of the orthodontic reference curve, design at least one set of dental orthodontic appliances based on a dental arch model data, the dental orthodontic appliance configured to set an orthodontic point of a tooth, and in a same dental arch coordinate system, the orthodontic point is located on a side of a feature point of a corresponding tooth in the dental arch model closer to the orthodontic reference curve.

22. An electronic device, **characterized by** comprising a processor, a memory, and a communication bus, wherein the processor and the memory communicate with each other through the communication bus;
the memory, configured to store an application; and
the processor, configured to, when executing the application stored on the memory, implement steps of a method for determining an orthodontic reference curve according to any one of claims 1-12, or implement steps of a method for designing a dental orthodontic appliance according to any one of claims 13-15.
